(19) Europäisches Patentamt European Patent Office Office européen des brevets

(11) **EP 3 928 689 B1**

(12) **EUROPEAN PATENT SPECIFICATION**

(45) Date of publication and mention of the grant of the patent:
**29.01.2025   Bulletin 2025/05**

(21) Application number: **20182503.1**

(22) Date of filing: **26.06.2020**

(51) International Patent Classification (IPC):
*A61B 5/02* (2006.01)     *A61B 5/026* (2006.01)
*A61B 5/0295* (2006.01)     *A61B 5/00* (2006.01)
*A61B 5/1455* (2006.01)

(52) Cooperative Patent Classification (CPC):
**A61B 5/02007; A61B 5/0261; A61B 5/0295;**
**A61B 5/14551; A61B 5/4035; A61B 5/7203;**
A61B 5/6826; A61B 2560/0223

(54) **APPARATUS AND METHOD FOR COMPENSATING ASSESSMENT OF PERIPHERAL ARTERIAL TONE**

VORRICHTUNG UND VERFAHREN ZUR KOMPENSATION DER BEURTEILUNG DES PERIPHEREN ARTERIELLEN TONUS

APPAREIL ET PROCÉDÉ DE COMPENSATION D'ÉVALUATION DU TONUS ARTÉRIEL PÉRIPHÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.12.2021   Bulletin 2021/52**

(73) Proprietor: **Ectosense NV**
**3110 Rotselaar (BE)**

(72) Inventors:
• **Geeurickx, Wout**
**3010 Kessel-Lo (BE)**
• **Massie, Frederik**
**3000 Leuven (BE)**
• **Vits, Steven**
**1060 Sint-Gillis (BE)**
• **Van Pee, Bart**
**3000 Leuven (BE)**

(74) Representative: **IP HILLS NV**
**Bellevue 5/501**
**9050 Gent-Ledeberg (BE)**

(56) References cited:
**US-A1- 2006 009 700     US-A1- 2009 326 353**
**US-A1- 2019 343 406**

**Description**

**Technical Field**

The present disclosure generally relates, amongst others, to a method and an apparatus for assessing Peripheral Arterial Tone or PAT. More particularly, it relates to robustly monitoring peripheral arterial tone for detecting for example sleep-related events.

**Background**

During a cardiac cycle of an individual, i.e. the period between two heart beats, blood is pumped through the vascular system of the individual in a pulsating fashion. In other words, during a cardiac cycle, the volume of blood in for example a finger, a nostril, an ear, a forehead, the inside of a mouth, a toe, a wrist, an ankle, etc., increases and decreases cyclically. The blood comprised in arteries is called arterial blood. The blood comprised in veins is called venous blood.

A common method for measuring this fluctuation in blood volume is optical plethysmography, or photoplethysmography, during which an optical plethysmogram, or PhotoPlethysmoGram, or PPG, is used to detect changes in blood volume in the microvascular bed of the tissue. A PPG is often obtained by shining light from one or more light sources, such as for example LEDs, onto an investigated volume, and detecting collected light corresponding to the light being reflected or transmitted in the investigated volume on a sensor, wherein the sensor may for example comprise or correspond to a photodetector, such as for example a photodiode. The light sources and the sensor can for example be arranged on opposite sides of a finger of an individual, hereby allowing the measurement of a transmission mode PPG, or at the same side of a finger of the individual, hereby allowing the measurement of a reflectance mode PPG. With each cardiac cycle the heart pumps arterial blood to the investigated volume. The physical events corresponding to a change in arterial blood volume in the investigated volume for example in a cardiac cycle can be captured by optical plethysmography. For example, US20060009700A1 describes an apparatus and method for the non-invasive evaluation, detection, and monitoring of a physiological state or medical condition by assessing peripheral circulation. For example, US2019343406A1 describes a method for non-invasively measuring cardiac output, stroke volume, or both by collecting plethysmographic waveform data of a patient.

In addition to cyclic fluctuations in arterial blood volume, the arterial blood volume in the tissue is also influenced by the diameters of small arteries, or arterioles, in the investigated volume. These arterioles have muscular walls which can contract to reduce the diameter arterioles. In other words, when these arterioles contract and reduce in diameter, the volume of arterial blood comprised in the corresponding arterioles evidently reduces. Optical plethysmography is a measurement technique which can be used to monitor changes in the volume of arterial blood comprised in arterioles when these arterioles contract in diameter. Monitoring changes in the arterial blood volume with optical plethysmography therefore empirically provides information on relative changes in muscle tension or 'tone' of the smooth muscle tissue of the arterioles, also referred to as Peripheral Arterial Tone or PAT.

Because arterial blood flow to the investigated volume can be modulated by multiple other physiological phenomena, optical plethysmography can also be used to monitor breathing, hypovolemia, other circulatory conditions, and for example can be used for diagnosing sleep disorders. Sleep disorder diagnosis is a medical field wherein a patient's sleep is monitored during a certain time, e.g. one or more nights. Based on the monitoring, different sleep-related events may be identified such as for example apneic events, snoring, or limb movements.

The reuptake of breathing at the end of a sleep apnea usually coincides with an adrenaline release. Adrenaline is released in the bloodstream and binds to adrenergic receptors in the arterioles in the investigated volume, which triggers an increase in muscle tension of the arterioles resulting in a reduction of the arteriole diameter and a reduction of arterial blood volume in the investigated volume. Monitoring Peripheral arterial tone through for example optical plethysmography can therefore provide valuable information on the occurrence of sleep-related events such as for example sleep apnea.

Hemoglobin is the main molecule which scatters the light emitted onto the investigated volume during a measurement with optical plethysmography. Hemoglobin comprises oxygenated hemoglobin, also known as $HbO_2$, and deoxygenated hemoglobin, also known as Hb. The extent to which hemoglobin in the arterial blood is oxygenated is referred to as the oxygen saturation, or $SpO_2$. The degree of absorption and scattering of light due to HbOz is significantly different from that of Hb and additionally depends on the wavelength of the light used for optical plethysmography.

As a result, an optical plethysmogram is not only dependent on the volume of arterial blood in the investigated volume but is also dependent on the hemoglobin composition of that arterial blood volume, which is characterized by the oxygen saturation. In other words, when attempting to measure changes in the PAT monitored by optical plethysmography, this measurement can be greatly influenced by changes in the oxygen saturation of the arterial blood volume which is monitored. For example, during apnea, as less oxygen is supplied to the lungs, the proportion of HbOz in a arterial blood volume within the investigated volume of the individual drops.

WO98/04182 for example describes a method and an apparatus for the detection of medical conditions by monitoring peripheral arterial tone. As described for example in EP1534115A2, the apparatus of EP1534115A2 performs optical plethysmography relying on a wavelength for which the degree of absorption and scattering for this particular wavelength caused by the HbO2 and the degree of absorption and scattering for this particular wavelength caused by the Hb are identical. Such wavelength is known as isosbestic wavelength. The light intensities measured in EP1534115A2 by optical plethysmography when relying on this isosbestic wavelength are therefore less dependent on the hemoglobin composition of the arterial blood volume, in other words, on the value of the oxygen saturation.

The solution described for example in EP1534115A2 requires the use of three light sources to monitor PAT via optical plethysmography: two light sources which do not generate light at an isosbestic wavelength to estimate the oxygen saturation, and a third light source which generates light at an isosbestic wavelength to estimate peripheral arterial tone. The use of three light sources increases the device cost and its size compared to a system which comprises only two light sources.

## Summary

It is thus an object of embodiments of the present invention to propose a computer-implemented method and an apparatus which do not show the inherent shortcomings of the prior art. More specifically, it is an object of embodiments of the present invention to propose a method and an apparatus to accurately and robustly minimize the influence caused by changes in the hemoglobin composition of the monitored arterial blood volume on the optical measurement of peripheral arterial tone without the need to use an isosbestic wavelength.

The scope of protection sought for various embodiments of the invention is set out by the independent claims.

The embodiments and features described in this specification that do not fall within the scope of the independent claims, if any, are to be interpreted as examples useful for understanding various embodiments of the invention.

There is a need for eliminating the effect of changes in the concentration of oxygenated and deoxygenated hemoglobin on the optical measurement of peripheral arterial tone.

Amongst others, it is an object of embodiments of the invention to reduce the influence caused by changes in the hemoglobin composition of the monitored arterial blood volume on the measurement of peripheral arterial tone with optical plethysmography.

This object is achieved, according to a first example aspect of the present disclosure, by a computer-implemented method for assessing peripheral arterial tone, PAT, of an individual monitored by optical plethysmography, wherein the method comprises the steps of:

- obtaining:

  ○ an optical plethysmography signal measured at an investigated volume of the individual;
  ○ light intensities acquired by optical plethysmography at two or more points in time along the optical plethysmography signal;
  ○ an oxygen saturation estimate;
  ○ calibration data;

- determining a compensation function from the oxygen saturation estimate and the calibration data; wherein the compensation function is a function of the oxygen saturation estimate;
- determining a first function of the light intensities; and
- determining a ratio of the first function and the compensation function, thereby evaluating one or more changes in arterial blood volume in the investigated volume between the two or more points in time and hereby assessing PAT of the individual.

The computer-implemented method according to the present disclosure allows determining peripheral arterial tone in an accurate and robust manner. By obtaining an oxygen saturation estimate of an individual under monitoring using optical plethysmography, the hemoglobin composition of the arterial blood volume of the individual at the investigated volume being monitored by optical plethysmography can be determined or estimated. The computer-implemented method according to the present disclosure can therefore adjust, or in other words compensate, the optical plethysmography signal such that the light intensities more reliably reflect changes in the arterial blood volume under monitoring by minimizing the influence caused by changes in the hemoglobin composition of the monitored arterial blood volume. In other words, the computer-implemented method according to the present disclosure reduces the effect on the optical plethysmography signal of changes in the hemoglobin composition of the arterial blood volume, and thereby minimizes or reduces the effect of changes in the hemoglobin composition of the arterial blood volume on the optical measurement of peripheral arterial tone. The computer-implemented method indeed determines a compensation function which is a function of the oxygen saturation estimate and divides a function of the light intensities measured by optical plethysmography by the compensation function for this oxygen saturation estimate. The resulting evaluation of a change in arterial blood volume in the investigated volume therefore provides a more accurate and robust assessment of peripheral arterial tone of the individual.

The computer-implemented method does not rely on the use of a light source emitting light at an isosbestic wavelength. On the contrary, the computer-implemented method is compatible with any usual setup for optical plethysmography comprising for example two light sources, wherein the two light sources for example emit light of two different wavelengths, such as for example one red wavelength and another infra-red wavelength. In other words, the computer-implemented method accurately measures changes in arterial blood volume in an investigated volume with just two light sources, which is the minimum number of light sources needed to determine the oxygen saturation estimate. Peripheral arterial tone is then mathematically derived from the optical plethysmography measurement with two light sources. This allows miniaturization compared to a system with three light sources and further allows cost optimization by eliminating the need for a third light source.

In the context of the present disclosure, an investigated volume of an individual is for example a volume defined in an investigated tissue of the individual which is monitored by optical plethysmography and in which light emitted by optical plethysmography propagates and is collected on a sensor for optical plethysmography. In other words, an investigated volume of an individual is for example a volume defined in an investigated tissue of the individual for which an optical plethysmography signal is acquired. For example, the investigated volume is a peripheral tissue volume of the individual. For example, the investigated volume is a volume defined in finger, a tip of a finger, a distal end of a digit of the individual, a nostril, an ear, a forehead, the inside of a mouth, a toe, a tip of a toe, a wrist, an ankle of the individual. In the context of the present disclosure, the investigated volume of an individual comprises the skin of the individual comprised in the investigated volume and further comprises the arterial blood volume present in the investigated volume. In the context of the present disclosure, peripheral arterial tone is understood as arterial tone changes in investigated arterial beds in the investigated volume of an individual. In other words, determining pulsatile volume changes in the vascular beds of the investigated volume of the individual allows determining or assessing information indicative for muscle tension or 'tone' of the smooth muscle tissue of the arterioles in the investigated volume and therefore allows determining or assessing peripheral arterial tone which is modulated by the sympathetic nervous system. Determining peripheral arterial tone is noninvasive and can for example be used to detect heart diseases, erectile dysfunction, sleep apnea, obstructive sleep apnea, cardiovascular conditions, etc..

In the context of the present disclosure, an optical plethysmography signal is a signal measured by optical plethysmography. For example, the optical plethysmography signal is an optical plethysmogram. For example, the optical plethysmography signal is a PPG. The optical plethysmography signal is for example measured at the tip of a finger of the individual by an optical plethysmography setup comprising at least two light sources and a sensor. In the context of the present disclosure, a light intensity corresponds to the intensity of light collected on the sensor of the optical plethysmography setup, wherein the light collected on the sensor corresponds to light generated by one or the two light sources being transmitted through or reflected in the investigated volume of the individual.

The blood comprised in arteries is called arterial blood. The blood comprised in veins is called venous blood. In the context of the present disclosure, the oxygen saturation estimate or $SpO_2$ or hemoglobin composition corresponds to a fraction of oxygenated hemoglobin related to a total amount of hemoglobin in the arterial blood volume. For example, the oxygen saturation estimate or $SpO_2$ or hemoglobin composition corresponds to a ratio of the concentration of oxygenated hemoglobin on the sum of the concentrations of oxygenated and deoxygenated hemoglobin in the arterial blood volume being monitored in the investigated volume. Alternatively, the oxygen saturation estimate or $SpO_2$ or hemoglobin composition corresponds to a ratio of the volume fraction of oxygenated hemoglobin on the sum of the volume fractions of oxygenated and deoxygenated hemoglobin in the arterial blood volume being monitored in the investigated volume.

In the context of the present disclosure, deoxygenated hemoglobin is defined as the form of hemoglobin without the bound oxygen, and without any other bound molecule such as for example carbon monoxide, carbon dioxide, or iron. In the context of the present disclosure, oxygenated hemoglobin is defined as the form of hemoglobin with the bound oxygen. In the context of the invention, light emitted by the light sources of an optical plethysmography setup comprises photons which reach the sensor through a probabilistic path of one or multiple scattering events. This optical path is not straight and is often assumed to follow a curved spatial probability distribution. The investigated volume along this curved optical path forms the volume which is sampled or investigated by optical plethysmography. In the context of the invention, a change in arterial blood volume in the investigated volume between the two points in time corresponds to a relative change between the volume of arterial blood present in the investigated volume at the first point in time and the volume of arterial blood present in the investigated volume at the second point in time.

According to example embodiments, the calibration data comprises predetermined calibration coefficients and/or predefined coefficients; and:

- the determining the compensation function corresponds to deriving the compensation function from the predefined coefficients; or

- the determining the compensation function corresponds to determining the predetermined calibration coefficients by fitting the oxygen saturation estimate to a calibration ratio.

In the context of the present disclosure, the predefined coefficients are for example known or can be determined from literature, such as for example from scientific publications. For example, the predefined coefficients comprise one or more extinction coefficients of chromophores and/or one or more absorption coefficients of chromophores and/or one or more scattering coefficients of chromophores. In the context of the present disclosure, a chromophore is a molecular unit which absorbs or scatters light in the investigated volume. For example, in the context of the present disclosure, examples of chromophores are melanin molecules, oxygenated hemoglobin, deoxygenated hemoglobin, etc. In the investigated volume, the decay in light intensity of the incident light emitted by a light source of an optical plethysmography setup follows the Beer-Lambert law which can be formulated as in equation (1):

$$I = I_0 e^{-d \sum_i \varepsilon_i V_i - G(\lambda)} \tag{1}$$

wherein:

- $I_0$ corresponds to the intensity of incident light emitted by a light source of an optical plethysmography setup;
- $\varepsilon_i$ corresponds to an extinction coefficient of chromophore $i$;
- $V_i$ corresponds to either a volume fraction or a concentration of chromophore i in the investigated volume;
- d corresponds to the optical path length, wherein the optical path length corresponds to the path length a photon travels along before reaching the sensor of an optical plethysmography setup, wherein the optical path length is a function of, among others, the wavelength of the incident light and of chromophore composition in the investigated volume, and wherein the optical path length is dependent on the distance between the light source emitting the photon and the sensor. From the publication of Chatterjee et al. entitled "Monte Carlo Analysis of Optical Interactions in Reflectance and Transmittance Finger Photoplethysmography", published in Sensors (Basel) 19(4):789 on February 15 2019, doi:10.3390/s19040789, for a distance between the light source emitting the photons and the sensor of a few millimetres, such as for example 3 mm or less than 3mm, it can be approximated that the optical path length can be assumed constant;
- G corresponds to a scattering dependent light intensity loss parameter and which depends on the wavelength $\lambda$ of the incident light emitted by the light source.

Considering the following parameters:

- $V_{i,d}$ corresponding to either a volume fraction or a concentration of chromophore i in the investigated volume at a first point in time along the optical plethysmography signal;
- $V_{i,s}$ corresponding to either a volume fraction or a concentration of chromophore i in the investigated volume at a second point in time along the optical plethysmography signal;
- $I_h$ corresponding to the light intensity measured by the sensor of the optical plethysmography setup at the first point in time;
- $I_l$ corresponding to the light intensity measured by the sensor of the optical plethysmography setup at the second point in time;

the Beer-Lambert law as formulated in equation (1) can be evaluated at the first point in time and at the second point in time. When taking a ratio of both expressions, equation (2) is obtained:

$$\frac{I_l}{I_h} = e^{-d \sum_i \varepsilon_i (V_{i,s} - V_{i,d})} \tag{2}$$

Then, equation (3) is obtained by taking the natural logarithm of both sides of equation (2), as follows:

$$ln\left(\frac{I_l}{I_h}\right) = -d \sum_i \varepsilon_i (V_{i,s} - V_{i,d}) \tag{3}$$

If a logarithm with another base $b$ than Euler's number e is used, equation (3) then becomes:

$$log_b\left(\frac{I_l}{I_h}\right) = \frac{-d\sum_i \varepsilon_i (V_{i,s} - V_{i,d})}{ln(b)} \tag{3'}$$

It can be seen that the above equation (3') is equal to equation (3) up to a constant $\frac{1}{ln(b)}$.

Equation (3) can be simplified to equation (4) when when the difference $V_{i,s} - V_{i,d}$ is being renamed $\Delta V_i$, thereby obtaining:

$$ln\left(\frac{I_l}{I_h}\right) = -d\sum \varepsilon_i \Delta V_i \tag{4}$$

From equation (4), it can be seen that the logarithm of the fraction of light intensities at the first point in time and at the second point in time is linearly related to the difference in the volume fraction or concentration of chromophore i between the first point in time and the second point in time.

Some chromophores remain attached to the epidermis of the individual between the two points in time along the optical plethysmography signal. For example, melanin molecules remain fixed to the investigated volume between the two points in time along the optical plethysmography signal. The difference in the volume fraction or concentration of such chromophores, such as for example melanin molecules, between the two points in time is therefore null. The contribution to the right-hand side of equation (4) of such chromophores is therefore null.

The main chromophores of which the volume fraction or concentration fluctuates between the two points in time along the optical plethysmography signal are the oxygenated and deoxygenated hemoglobin in the arterial blood volume. The two predominant forms of hemoglobin in the context of the present disclosure, i.e. oxygenated hemoglobin and deoxygenated hemoglobin, demonstrate absorption and scattering coefficients which are significantly different for most wavelengths of light.

The effect of all other chromophores wherein all other chromophores are not oxygenated hemoglobin nor deoxygenated hemoglobin, of which the volume fraction or concentration fluctuates between the two points in time along the optical plethysmography signal can be written as the product of their combined extinction coefficient, $\varepsilon_{other}$ and one minus the sum of the volume fractions or concentrations of oxygenated hemoglobin and deoxygenated hemoglobin, wherein the sum of all volume fractions or concentrations equals 1.

Taking the above considerations into account, equation (4) can then be rewritten as follows into equation (5):

$$ln\left(\frac{I_l}{I_h}\right) = -d\left(\varepsilon_{HbO_2}\Delta V_{HbO_2} + \varepsilon_{Hb}\Delta V_{Hb}\right.$$

$$\left. + \varepsilon_{other}\left(1 - \left(V_{Hb,s} + V_{HbO_2,s}\right) - \left(1 - \left(V_{Hb,d} + V_{HbO_2,d}\right)\right)\right)\right)$$

$$ln\left(\frac{I_l}{I_h}\right) = -d\left(\varepsilon_{HbO_2}\Delta V_{HbO_2} + \varepsilon_{Hb}\Delta V_{Hb} + \varepsilon_{other}\left(-(\Delta V_{Hb} + \Delta V_{HbO_2})\right)\right)$$

$$ln\left(\frac{I_l}{I_h}\right) = -d\left(\varepsilon_{HbO_2}\Delta V_{HbO_2} + \varepsilon_{Hb}\Delta V_{Hb} - \varepsilon_{other}\left(\Delta V_{Hb} + \Delta V_{HbO_2}\right)\right)$$

$$ln\left(\frac{I_l}{I_h}\right) = -d\left((\varepsilon_{HbO_2} - \varepsilon_{other})\Delta V_{HbO_2} + (\varepsilon_{Hb} - \varepsilon_{other})\Delta V_{Hb}\right) \tag{5}$$

It is known that the oxygen saturation estimate can be defined according to equation (6):

$$\frac{V_{HbO_2}}{V_{HbO_2} + V_{Hb}} = SpO_2 \tag{6}$$

wherein:

- $V_{HbO_2}$ corresponds to either the volume fraction or the concentration of oxygenated hemoglobin comprised within the arterial blood in the investigated volume;
- $V_{Hb}$ corresponds to either the volume fraction or the concentration of deoxygenated hemoglobin comprised within the arterial blood in the investigated volume.

Additionally, $V_{blood}$ is defined as the total volume fraction or the total concentration of oxygenated and deoxygenated hemoglobin comprised within the arterial blood in the investigated volume and is defined as follows in equation (7):

$$V_{HbO_2} + V_{Hb} = V_{blood} \qquad (7)$$

It is assumed that under normal circumstances, the sum of the volume fractions or concentrations of oxygenated and deoxygenated hemoglobin within the arterial blood volume or the sum of the concentrations of oxygenated and deoxygenated hemoglobin within the arterial blood volume remains approximately constant throughout the measurement of the optical plethysmography signal. Indeed, only the ratio of oxygenated hemoglobin and deoxygenated hemoglobin, i.e. only the oxygen saturation estimate, might significantly change during the monitoring of the individual by optical plethysmography, for example during sleep apnea.

From equations (6) and (7), the following can be obtained:

$$SpO_2 V_{blood} = V_{HbO_2}$$

$$(1 - SpO_2)V_{blood} = V_{Hb} \qquad (8)$$

By substituting expression (8) into equation (5), the following can be obtained:

$$ln\left(\frac{I_l}{I_h}\right) = -d\left(\left(\varepsilon_{HbO_2} - \varepsilon_{other}\right)SpO_2\, \Delta V_{blood} + \left(\varepsilon_{Hb}\right.\right.$$
$$\left.\left. - \varepsilon_{other}\right)(1 - SpO_2)\Delta V_{blood}\right) \qquad (9)$$

Two predetermined calibration coefficients $Q_1$ and $Q_2$, which are two constants, can be defined as follows:

$$Q_1 = \varepsilon_{HbO_2} - \varepsilon_{Hb}$$

$$Q_2 = \varepsilon_{Hb} - \varepsilon_{other}$$

After rewriting equation (9) in view of the two predetermined calibration coefficients $Q_1$ and $Q_2$, equation (10) can be obtained, wherein the left-hand side of equation (10) corresponds to the evaluation function, and wherein $Q_1 SpO_2 + Q_2$ corresponds to the compensation function which is a function of the oxygen saturation estimate:

$$ln\left(\frac{I_l}{I_h}\right) = -d\Delta V_{blood}(Q_1 SpO_2 + Q_2) \qquad (10)$$

Equation (11) can therefore be obtained:

$$\frac{ln\left(\frac{I_l}{I_h}\right)}{(Q_1 SpO_2 + Q_2)} = -d\Delta V_{blood} \qquad (11)$$

Relation (12) is therefore deducted from equation (11):

$$\frac{ln\left(\frac{I_l}{I_h}\right)}{(Q_1 SpO_2 + Q_2)} \sim \Delta V_{blood} \qquad (12)$$

**EP 3 928 689 B1**

Relation (12) highlights a term on the left-hand side which shows a linear relationship with the change $\Delta V_{blood}$ in total volume fraction or concentration of oxygenated and deoxygenated hemoglobin in the investigated volume between the first and the second points in time. Under the assumption of a constant volume fraction or concentration of the sum of oxygenated and deoxygenated hemoglobin in the in the arterial blood, $\Delta V_{blood}$ is a linear proxy for arterial blood volume fluctuations within the investigated volume. A change in arterial blood volume in the investigated volume is thus evaluated when determining a ratio of a function of the light intensities collected on the sensor when measuring with optical plethysmography and of a compensation function which is a function of the oxygen saturation estimate.

According to example embodiments, at least one of the points in time corresponds to the diastole in a cardiac cycle of the individual and/or wherein at least one of the points in time corresponds to the systole in a cardiac cycle of the individual.

During systole, the volume of arterial blood in the investigated volume of the individual is maximum, resulting in the largest absorption and scattering of light of any point in time within a cardiac cycle, i.e. the period between two heart beats, since hemoglobin is one of the main absorbers and scatters of photons in the investigated volume, hence resulting in the lowest measured light intensity on the sensor of the optical plethysmography setup. Conversely, during diastole, the volume of arterial blood in the investigated volume of the individual is minimum, resulting in the lowest absorption and scattering of light of any point in time within a cardiac cycle and hence highest measured light intensity on the sensor of the optical plethysmography setup. At least one first point in time corresponds for example to the diastole in a first cardiac cycle and/or at least one second point in time corresponds for example to the systole in a second cardiac cycle different from the first cardiac cycle. Alternatively, at least one first point in time corresponds for example to the systole in a first cardiac cycle and/or at least one second point in time corresponds for example to the diastole in a second cardiac cycle different from the first cardiac cycle. Alternatively, at least one first point in time corresponds for example to the systole or to the diastole in a cardiac cycle and at least one second point in time corresponds to any point in time within the same cardiac cycle or within a different cardiac cycle.

According to example embodiments, at least two of the two or more points in time are within one cardiac cycle of the individual.

From equation (4), the logarithm of the fraction of the light intensities at systole and diastole is linearly related to the changes in the chromophore volume fraction or concentration between systole and diastole. In order to achieve the highest signal to noise ration during the measurement with the optical plethysmography setup, it is best to measure at at least two different points in time within one cardiac cycle, wherein the at least two points in time demonstrate a large difference in light intensity on the sensor, and as a result, a large difference in arterial blood volume. The two points in time which correspond to this largest difference are typically at diastole and at systole within one cardiac cycle.

According to example embodiments, the method further comprises the steps of determining an evaluation function which is a function of the light intensities; and wherein the determining a ratio corresponds to determining a ratio of the evaluation function and the compensation function.

According to equation (3), the evaluation function corresponds to the natural logarithm of a function of the light intensities. Alternatively, starting from equation (2), any other evaluation function defined as a function of the light intensities could be used, for example a linear approximation of the natural logarithm of a function of the light intensities, or for example a Taylor series approximation of a function of the light intensities, or for example a linear approximation of other base logarithms of a function of the light intensities. The change in arterial blood volume in the investigated volume between the two or more points in time, and hereby peripheral arterial tone, are then assessed by determining a ratio of the evaluation function and of the compensation function.

According to example embodiments, the evaluation function corresponds to a logarithm of a function of the light intensities.

According to equation (3), the evaluation function corresponds to the natural logarithm of a function of the light intensities. Alternatively, starting from equation (2), any other evaluation function defined as a function of the light intensities could be used, for example a linear approximation of the logarithm of a function of the light intensities, or for example a Taylor series approximation of a function of the light intensities, or for example a linear approximation of other base logarithms of a function of the light intensities. Alternatively, the evaluation function corresponds approximately to the ratio of the pulsatile waveform or AC component of the optical plethysmography signal and of the slowly varying baseline or DC component of the optical plethysmography signal, resulting in equation (13):

$$ln\left(\frac{I_h}{I_l}\right) = ln\left(\frac{I_l}{I_l} + \frac{I_h - I_l}{I_l}\right) = ln\left(1 + \frac{I_h - I_l}{I_l}\right) \cong \frac{I_h - I_l}{I_l} \equiv \frac{AC_{ppg}}{DC_{ppg}} \qquad (13)$$

According to example embodiments, the evaluation function corresponds to a logarithm of a ratio of the light intensities; and wherein the evaluation function depends on one or more of the following:

8

- a function of the oxygen saturation estimate;
- the changes in arterial blood volume in the investigated volume.

The left-hand side of equation (10) therefore corresponds to the evaluation function, i.e. to the logarithm of a ratio of the light intensities measured by the sensor when investigating the investigated volume of the individual with optical plethysmography.

According to example embodiments, the method further comprises the steps of:

- providing a first light source configured to emit light at a first wavelength;
- providing a second light source configured to emit light at a second wavelength;
- providing a sensor;
- collecting, by optical plethysmography and on the sensor, propagated light corresponding to the light being transmitted or reflected when propagating in a distal end of a digit of the individual at the two or more points in time;
- for the first wavelength, determining first light intensities of the propagated light on the sensor at the two or more points in time;
- for the second wavelength, determining second light intensities of the propagated light on the sensor at the two or more points in time;
- determining a first ratio corresponding to a ratio of the first light intensities at the first wavelength;
- determining a second ratio corresponding to a ratio of the second light intensities at the second wavelength; and
- fitting the oxygen saturation estimate to the calibration ratio thereby determining the predetermined calibration coefficients.

The oxygen saturation estimate can be obtained for example from a reference measurement obtained by optical plethysmography. Alternatively, the oxygen saturation estimate can be obtained for example by any other suitable apparatus or method which measures oxygen saturation near the investigated volume being assessed. The evaluation function can for example be measured by the sensor of the optical plethysmography setup. Using the light intensities collected by optical plethysmography for the first wavelength $\lambda_1$ and the light intensities collected by optical plethysmography for the second wavelength $\lambda_2$, it becomes possible to determine the predetermined calibration coefficients $Q_{1,\lambda_1}$ and $Q_{2,\lambda_1}$ of the compensation function for the first wavelength $\lambda_1$ and the predetermined calibration coefficients $Q_{1,\lambda_2}$ and $Q_{2,\lambda_2}$ of the compensation function for the first wavelength $\lambda_2$ by calculating the following equation (14):

$$\frac{ln\left(\frac{I_l}{I_h}\right)_{\lambda 1}}{ln\left(\frac{I_l}{I_h}\right)_{\lambda 2}} = \frac{-d\Delta V_{blood}\left(Q_{1,\lambda_1}SpO_2 + Q_{2,\lambda_1}\right)}{-d\Delta V_{blood}\left(Q_{1,\lambda_2}SpO_2 + Q_{2,\lambda_2}\right)} \tag{14}$$

which simplifies into equation (15):

$$\frac{ln\left(\frac{I_l}{I_h}\right)_{\lambda 1}}{ln\left(\frac{I_l}{I_h}\right)_{\lambda 2}} = \frac{\left(Q_{1,\lambda_1}SpO_2 + Q_{2,\lambda_1}\right)}{\left(Q_{1,\lambda_2}SpO_2 + Q_{2,\lambda_2}\right)} \tag{15}$$

The left-hand side of equation (15), i.e. the calibration ratio, can be computed from the measurements of the sensor of the optical plethysmography setup of both wavelengths $\lambda_1$ and $\lambda_2$.

Alternatively, to account for non-linearities that were not properly captured by the above discussed theory, equation (12) can be rewritten as follows in equation (16):

$$\frac{ln\left(\frac{I_l}{I_h}\right)}{f_{\lambda_1}(SpO_2)} \sim \Delta V_{blood} \tag{16}$$

wherein $f_{\lambda_1}$ is corresponds to the compensation function at the first wavelength $\lambda_1$, and which is a non-factorizable function of $SpO_2$.

Then equation (14) can be rewritten as follows in equation (17) in view of equation (16):

$$\frac{ln\left(\frac{I_l}{I_h}\right)_{\lambda 1}}{ln\left(\frac{I_l}{I_h}\right)_{\lambda 2}} = \frac{f_{\lambda 1}(SpO_2)}{f_{\lambda 2}(SpO_2)} \qquad (17)$$

wherein $f_{\lambda_2}$ is corresponds to the compensation function at the second wavelength $\lambda_2$.

Optical plethysmography technology uses a simple and noninvasive setup probe or biosensor. The optical plethysmography biosensor non-invasively measures pulsatile arterial volume changes in the investigated volume, and thereby assesses PAT, by collecting the optical plethysmography signal. The first and/or the second light sources are for example LEDs or any other suitable light sources which can be miniaturized to fit in the optical plethysmography biosensor. The first wavelength is for example different from the second wavelength. For example, the first wavelength and the second wavelength are comprised in the red spectrum. For example, the first wavelength is comprised in the red spectrum, and the second wavelength is comprised in the infra-red spectrum. A physical distance between the light sources and the sensor is for example a few millimeters, such as for example less than 3mm.

According to example embodiments, the method further comprises the steps of:

- collecting, by optical plethysmography and on the sensor, propagated light corresponding to light at the first wavelength or at the second wavelength being transmitted or reflected when propagating in the investigated volume of the individual at the two or more points in time; and
- determining the light intensities of the propagated light on the sensor at the two or more points in time.

According to example embodiments, the oxygen saturation estimate depends on a ratio of the concentration of oxygenated hemoglobin on a total concentration of oxygenated and deoxygenated hemoglobin in the arterial blood volume.

According to example embodiments, the method further comprises the step of determining the oxygen saturation estimate near the investigated volume of the individual, for example near the distal end of the digit of the individual.

According to example embodiments, the compensation function is derived from a regression which maps the oxygen saturation estimate onto the predetermined calibration coefficients.

Evaluating the compensation function corresponds to determining for example a least-squares regression which maps the oxygen saturation estimate onto the predetermined calibration coefficients. After gathering data in for example a clinical trial, it becomes possible to fit the oxygen saturation estimate to the calibration ratio on the left-side of equation (15) and thereby determining the predetermined calibration coefficients, thereby estimating the compensation function. In other words, a least-squares regression maps the oxygen saturation estimate onto $\dfrac{ln\left(\frac{I_l}{I_h}\right)_{\lambda 1}}{ln\left(\frac{I_l}{I_h}\right)_{\lambda 2}}$ of equation (15).

According to example embodiments, the method further comprises the step of forcing that the regression uses a first-degree rational mapping when evaluating the compensation function.

For example, the method further comprises the step of forcing that a least-squares regression uses a first-degree rational mapping when estimating the compensation function. After the least-squares regression maps the oxygen saturation estimate onto $\dfrac{ln\left(\frac{I_l}{I_h}\right)_{\lambda 1}}{ln\left(\frac{I_l}{I_h}\right)_{\lambda 2}}$ of equation (15), and by forcing that the linear regression uses a first degree rational mapping, i.e. a first degree polynomial in the numerator and a first degree polynomial in the denominator, the least-squares fit yields the best estimate of the predetermined calibration coefficients $Q_{1,\lambda_1}$, $Q_{1,\lambda_2}$, $Q_{2,\lambda_1}$, and $Q_{2,\lambda_2}$ up to a constant scalar. As the linear relationship between a measurable parameter and $\Delta V_{blood}$ is relevant, determination of the predetermined calibration coefficients up to a constant factor is sufficient.

Alternatively, referring to equation (17), compensation function $f_{\lambda_1}$ and compensation function $f_{\lambda_2}$ can be determined again through a least-squares regression which maps a measured reference oxygen saturation estimate onto a known right-hand side of equation (17). The only constraint on the regression is that the mapping makes use of a non-factorizable function of oxygen saturation estimate in the numerator and the denominator.

According to a second example aspect, an apparatus is disclosed, wherein the apparatus comprises at least one processor and at least one memory including computer program code, the at least one memory and computer program code configured to, with the at least one processor, cause the apparatus to perform:

- obtaining:

　　◦ an optical plethysmography signal measured at an investigated volume of the individual;
　　◦ light intensities acquired by optical plethysmography at two or more points in time along the optical plethysmography signal;
　　◦ an oxygen saturation estimate;
　　◦ calibration data;

- determining a compensation function from the oxygen saturation estimate and the calibration data; wherein the compensation function is a function of the oxygen saturation estimate;
- determining a first function of the light intensities; and
- determining a ratio of the first function and the compensation function, thereby evaluating one or more changes in arterial blood volume in the investigated volume between the two or more points in time and hereby assessing PAT of the individual.

The computer-implemented method according to the present disclosure allows determining peripheral arterial tone in an accurate and robust manner. By obtaining an oxygen saturation estimate of an individual under monitoring using optical plethysmography, the hemoglobin composition of the arterial blood volume of the individual at the investigated volume being monitored by optical plethysmography can be determined or estimated. The computer-implemented method according to the present disclosure can therefore adjust, or in other words compensate, the optical plethysmography signal measured by optical plethysmography such that the light intensities more reliably reflect changes in the arterial blood volume under monitoring by minimizing the influence caused by changes in the hemoglobin composition of the monitored arterial blood volume. In other words, the computer-implemented method according to the present disclosure reduces the effect on the optical plethysmography signal of changes in the hemoglobin composition of the arterial blood volume, and thereby minimizes or reduces the effect of changes in the hemoglobin composition of the arterial blood volume on the optical measurement of peripheral arterial tone. The computer-implemented method indeed determines a compensation function which is a function of the oxygen saturation estimate and divides a function of the light intensities measured by optical plethysmography by the compensation function for this oxygen saturation estimate. The resulting evaluation of a change in arterial blood volume in the investigated volume therefore provides a more accurate and robust assessment of peripheral arterial tone of the individual.

The apparatus does not rely on the use of a light source emitting light at an isosbestic wavelength. The apparatus is compatible with any usual setup for optical plethysmography comprising for example two light sources, wherein the two light sources for example emit light of two different wavelengths, such as for example one red wavelength and another infra-red wavelength. In other words, the apparatus accurately measures changes in arterial blood volume in an investigated volume through measurement setups with just two light sources, which is the minimum number of light sources needed to determine the oxygen saturation estimate. Peripheral arterial tone is then mathematically derived by the apparatus from the optical plethysmography measurement with two light sources. This allows miniaturization compared to an optical plethysmography system comprising three light sources and further allows cost optimization by eliminating the need for a third light source.

According to example embodiments, a system is provided, wherein the system comprises an apparatus according to a second example aspect of the invention, and further comprises:

- a light source configured to emit light; and
- a sensor configured to collect by optical plethysmography propagated light corresponding to the light being transmitted or reflected when propagating in the investigated volume of the individual at the two or more points in time; and further configured to determine the light intensities of the propagated light at the two or more points in time.

The sensor collects propagated light by optical plethysmography, wherein the propagated light corresponds to the light being transmitted or reflected when propagating in the investigated volume of the individual, such as for example a distal end of a digit of the individual, at the two or more points in time. The system further optionally comprises a wireless transmitter comprising a wireless communication interface, wherein the wireless transmitter is configured to transmit the determined peripheral arterial tone wirelessly for further processing by the apparatus. The wireless communication interface is preferably a low power communication interface, e.g. a Bluetooth Low Energy, BLE, wireless interface.

According to a third example aspect, a computer program product comprising computer-executable instructions for causing a system to perform at least the following is provided:

- obtaining:

　　◦ an optical plethysmography signal measured at an investigated volume of the individual;
　　◦ light intensities acquired by optical plethysmography at two or more points in time along the optical plethysmography signal;
◦ an oxygen saturation estimate;
◦ calibration data;

- determining a compensation function from the oxygen saturation estimate and the calibration data; wherein the compensation function is a function of the oxygen saturation estimate; and
- determining a ratio of a function of the light intensities and of the compensation function, thereby evaluating one or more changes in arterial blood volume in the investigated volume between the two or more points in time and hereby assessing PAT of the individual.

According to a fourth example aspect, a computer readable storage medium is provided, wherein the computer readable storage medium comprises computer-executable instructions for performing the following steps when the program is run on a computer:

- obtaining:

  ◦ an optical plethysmography signal measured at an investigated volume of the individual;
  ◦ light intensities acquired by a sensor of optical plethysmography at two or more points in time along the optical plethysmography signal;
  ◦ an oxygen saturation estimate;
  ◦ calibration data;

- determining a compensation function from the oxygen saturation estimate and the calibration data; wherein the compensation function is a function of the oxygen saturation estimate;
- determining a first function of the light intensities; and
- determining a ratio of the first function and the compensation function, thereby evaluating one or more changes in arterial blood volume in the investigated volume between the two or more points in time and hereby assessing PAT of the individual.

## Brief Description of the Drawings

Some example embodiments will now be described with reference to the accompanying drawings.

Fig. 1 depicts an example embodiment of an apparatus according to the present disclosure.

Fig. 2 depicts an example embodiment of a system according to the present disclosure comprising an apparatus according to the present disclosure.

Fig. 3 depicts an example embodiment of a system according to the present disclosure comprising an apparatus according to the present disclosure.

Figs. 4A and 4B depict an example embodiment of a calibration of an apparatus according to the present disclosure.

Fig. 5 depicts an example embodiment of a PAT measurement of an individual assessed by an apparatus according to the present disclosure

Fig. 6 depicts an example embodiment of a computer-implemented method according to the present disclosure.

Fig. 7 shows an example embodiment of a suitable computing system for performing one or several steps in embodiments of the invention.

## Detailed Description of Embodiment(s)

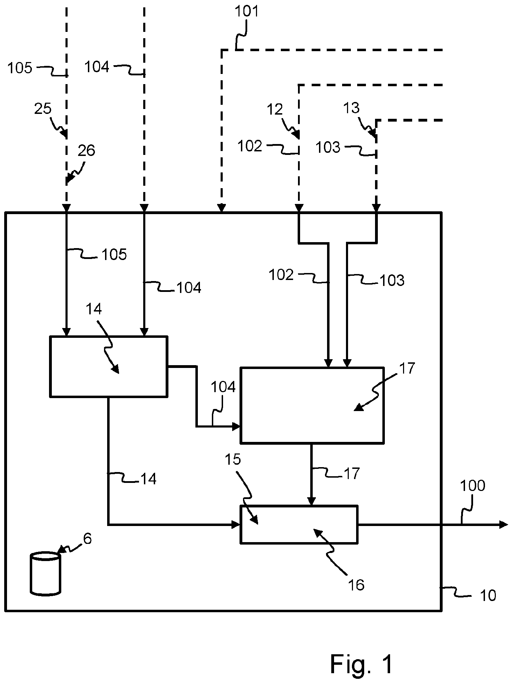

Fig. 1 illustrates an example embodiment of an apparatus 10 according to the present disclosure. The apparatus 10 comprises at least one memory 6, at least one processor, wherein the memory 6 includes computer program code configured to, with the at least one processor, cause the apparatus 10 to perform the following:

- obtain:

- an optical plethysmography signal 101 measured at an investigated volume 11 of an individual;
- light intensities 102;103 acquired by optical plethysmography at two or more points in time 12;13 along the optical plethysmography signal 101, wherein the light intensity 102 is respectively acquired at the point in time 12 and wherein the light intensity 103 is respectively acquired at the point in time 13;
  - an oxygen saturation estimate 104;
  - calibration data 105;

- determine a compensation function 14 from the oxygen saturation estimate 104 and the calibration data 105; wherein the compensation function 14 is a function of the oxygen saturation estimate 104;
- determine a first function of the light intensities 102; 103; and
- determining a ratio 15 of the first function and the compensation function 14, thereby evaluating one or more changes in arterial blood volume 16 in the investigated volume 11 between the two or more points in time 12;13 and hereby assessing PAT 100 of the individual.

The apparatus 10 obtains one or more of the optical plethysmography signal 101, light intensities 102;103, the oxygen saturation estimate 104, the calibration data 105 from an external device. According to an alternative embodiment, the apparatus 10 obtains one or more of the optical plethysmography signal 101, light intensities 102;103, the oxygen saturation estimate 104, the calibration data 105 from the memory 6. According to a further alternative embodiment, the apparatus 10 obtains one or more of the optical plethysmography signal 101, light intensities 102; 103, the oxygen saturation estimate 104, the calibration data 105 from the memory 6 and/or from an external device. The calibration data 105 comprises predetermined calibration coefficients 25 and/or predefined coefficients 26. The apparatus 10 is then further configured to determine the compensation function 14 by deriving the compensation function 14 from the predefined coefficients 26. According to an alternative embodiment, the apparatus 10 is configured to determine the compensation function 14 by fitting the oxygen saturation estimate 104 to a calibration ratio, thereby obtaining the predetermined calibration coefficients 25 and thereby estimating the compensation function 14. Optionally, at least one point in time 12 corresponds to the diastole of a cardiac cycle of the individual and/or at least one point in time 13 corresponds to the systole of a cardiac cycle of the individual. The apparatus 10 is configured to determine an evaluation function 17 which is a function of the light intensities 102;103. The apparatus then determines a ratio 15 corresponding to a ratio of the evaluation function 17 and of the compensation function 14. The evaluation function 17 for example corresponds to a logarithm of a function of the light intensities 102;103. The evaluation function 17 for example corresponds to a logarithm of a ratio of the light intensities 102;103. The evaluation function 17 depends on one or more of the following: an optical path length, a function of the oxygen saturation estimate, a change in arterial blood volume in the investigated volume.

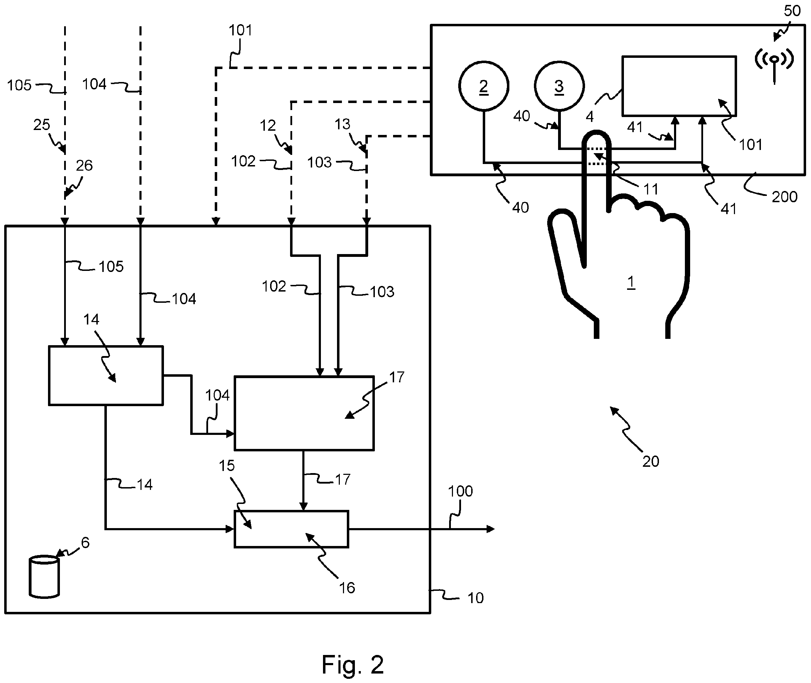

Fig. 2 illustrates an example embodiment of a system 20 according to the present disclosure. Components having identical reference numbers to numbers on Fig. 1 perform the same function. The system 20 of Fig. 2 comprises an apparatus 10 according to the present disclosure. Optionally, the system 20 further comprises a light source 2;3 and a sensor 4. A light source 2;3 is configured to emit light 40. The apparatus 10 is configured to:

- obtain:

  - an optical plethysmography signal 101 measured at an investigated volume 11 of an individual 1;
  - light intensities 102;103 acquired by a sensor 4 of optical plethysmography at two or more points in time 12;13 along the optical plethysmography signal 101, wherein the light intensity 102 is respectively acquired at the point in time 12 and wherein the light intensity 103 is respectively acquired at the point in time 13;
  - an oxygen saturation estimate 104;
  - calibration data 105;

- determine a compensation function 14 from the oxygen saturation estimate 104 and the calibration data 105; wherein the compensation function 14 is a function of the oxygen saturation estimate 104;
- determining a first function of the light intensities 102;103; and
- determining a ratio 15 of the first function and the compensation function 14, thereby evaluating one or more changes in arterial blood volume 16 in the investigated volume 11 between the two or more points in time 12;13 and hereby assessing PAT 100 of the individual 1.

The apparatus 10 obtains one or more of the optical plethysmography signal 101, light intensities 102;103, the oxygen saturation estimate 104, the calibration data 105 from an external device 200 comprising the light sources 2;3 and/or the sensor 4. For example, the external device 200 determines the arterial blood volume pulse in the investigated volume 11 of the individual 1. The external device 200 comprises a battery for powering the different electrical components 2;3;4. The light sources 2;3 are configured to emit light, i.e. to transmit the light 40 into the investigated volume 11 of the individual attached to the external device 200, for example into the finger 11 of the individual 1 as illustrated, more particularly to a distal end 11 of a finger of the individual. The external device 200 further comprises control circuitry for controlling the light sources 2;3, i.e., for enabling or disabling the light sources 2;3 and for receiving the measured arterial blood volume pulse values from the sensor 4. Control circuitry may further comprise a memory component for temporarily storing the obtained measurements. Control circuitry is further coupled to a wireless interfacing circuitry 50 and configured to forward the measurements to the wireless interfacing circuitry 50. Wireless interface 50 may support a short range and/or low power wireless communication protocol for efficient transmission of the measurements to a receiving part of the system. Wireless interface 50 may for example operate according to the Bluetooth Low Energy, BLE, protocol as defined by the Bluetooth Special Interest Group or according to a Near Field Communication, NFC, protocol. Operation by such protocols together with forwarding of the raw optical plethysmography signal 101 allows miniaturization of the external device 200 such that it fits on a finger or a nostril and allows operation during multiple nights. According to an alternative embodiment, the apparatus 10 obtains one or more of the optical plethysmography signal 101, light intensities 102;103, the oxygen saturation estimate 104, the calibration data 105 from the memory 6. According to a further alternative embodiment, the apparatus 10 obtains one or more of the optical plethysmography signal 101, light intensities 102;103, the oxygen saturation estimate 104, the calibration data 105 from the memory 6 and/or from an external device comprising the light sources 2;3 and/or the sensor 4. The calibration data 105 comprises predetermined calibration coefficients 25 and/or predefined coefficients 26. The apparatus 10 is then further configured to determine the compensation function 14 by deriving the compensation function 14 from the predefined coefficients 26. According to an alternative embodiment, the apparatus 10 is configured to determine the compensation function 14 by fitting the oxygen saturation estimate 104 to the predetermined calibration coefficients 25. Optionally, at least one point in time 12 corresponds to the diastole of a cardiac cycle of the individual and/or at least one point in time 13 corresponds to the systole of a cardiac cycle of the individual. The apparatus 10 is configured to determine an evaluation function 17 which is a function of the light intensities 102;103. The apparatus 10 collects, by optical plethysmography on the sensor 4, propagated light 41 corresponding to light 40 emitted by the first light source 2 or by the second light source 3, wherein the light 40 is transmitted or reflected by the investigated volume when propagating in the distal end of the digit of the individual at the two or more points in time 12;13 along the optical plethysmography signal 101. In other words, the apparatus 10 collects, by optical plethysmography on the sensor 4, a light intensity 102 corresponding to propagated light 41 corresponding to light 40 emitted by the first light source 2 or by the second light source 3, wherein the light 40 is transmitted or reflected by the investigated volume 11 when propagating in the distal end of the digit of the individual 1 and is collected on the sensor 4 at a first point in time 12; and the apparatus 10 collects, by optical plethysmography on the sensor 4, a light intensity 103 corresponding to propagated light 41 corresponding to light 40 emitted by the same light source 2 or 3, wherein the light 40 is transmitted or reflected by the investigated volume 11 when propagating in the distal end of the digit of the individual 1 and is collected on the sensor 4 at a second point in time 13. The apparatus then determines a ratio 15 corresponding to a ratio of the evaluation function 17 and of the compensation function 14. The evaluation function 17 for example corresponds to a logarithm of a function of the light intensities 102;103. The evaluation function 17 for example corresponds to a logarithm of a ratio of the light intensities 102;103. The evaluation function 17 depends on one or more of the following: an optical path length, a function of the oxygen saturation estimate, a change in arterial blood volume in the investigated volume. The apparatus 101 optionally determines the oxygen saturation estimate 104 near the distal end of the digit of the individual 1. Evaluating the compensation function 14 optionally corresponds to determining a regression which maps the oxygen saturation estimate 104 onto the predetermined calibration coefficients 25. The apparatus 10 optionally forces that the regression uses a first-degree rational mapping when evaluating the compensation function 14.

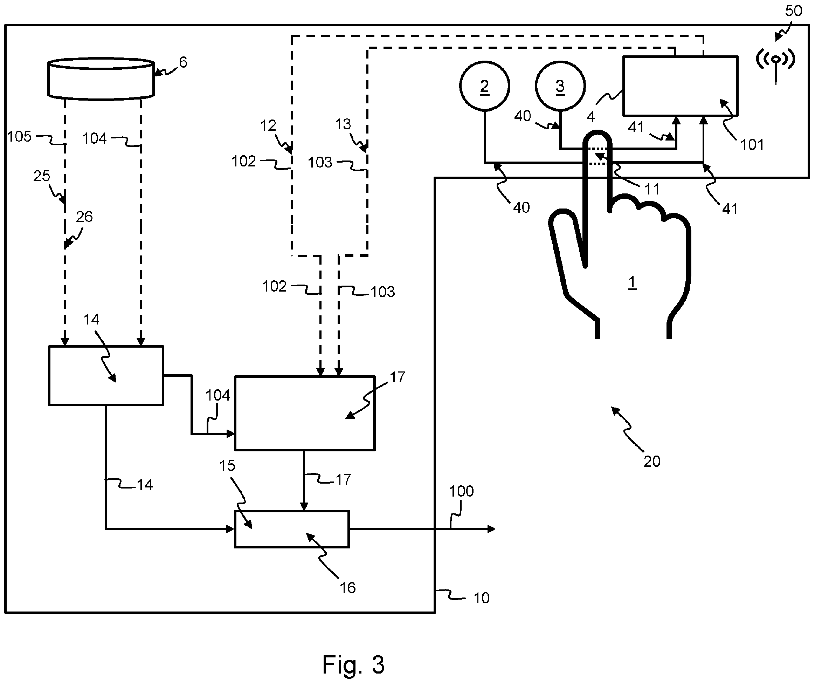

Fig. 3 illustrates an example embodiment of a system 20 according to the present disclosure. Components having identical reference numbers to numbers on Fig. 1 or Fig. 2 perform the same function. The system 20 of Fig. 3 comprises an apparatus 10 according to the present disclosure. Optionally, the system 20 further comprises a light source 2;3 and a sensor 4. A light source 2;3 is configured to emit light 40. The apparatus 10 is configured to:

- obtain:

    ◦ an optical plethysmography signal 101 measured at an investigated volume 11 of an individual 1;
    ◦ light intensities 102;103 acquired by a sensor 4 of optical plethysmography at two or more points in time 12;13 along the optical plethysmography signal 101, wherein the light intensity 102 is respectively acquired at the point in time 12 and wherein the light intensity 103 is respectively acquired at the point in time 13;
    ◦ an oxygen saturation estimate 104;
    ◦ calibration data 105;

- determine a compensation function 14 from the oxygen saturation estimate 104 and the calibration data 105; wherein the compensation function 14 is a function of the oxygen saturation estimate 104;

- determine a first function of the light intensities 102;103; and
- determining a ratio 15 of the first function and f the compensation function 14, thereby evaluating a change in arterial blood volume 16 in the investigated volume 11 between the two or more points in time 12;13 and hereby assessing PAT 100 of the individual 1.

The apparatus 10 obtains one or more of the optical plethysmography signal 101, light intensities 102;103, the oxygen saturation estimate 104, the calibration data 105 from the light sources 2;3 and/or the sensor 4. For example, the apparatus 10 determines the arterial blood volume pulse in the investigated volume 11 of the individual 1. The apparatus 10 comprises a battery for powering the different electrical components 2;3;4. The light sources 2;3 are configured to emit light, i.e. to transmit the light 40 into the investigated volume 11 of the individual attached to the apparatus 10, for example into the finger 11 of the individual 1 as illustrated, more particularly to a distal end 11 of a finger of the individual as illustrated. The apparatus 10 further comprises control circuitry for controlling the light sources 2;3, i.e., for enabling or disabling the light sources 2;3 and for receiving the measured arterial blood volume pulse values from the sensor 4. Control circuitry may further comprise a memory component for temporarily storing the obtained measurements. Control circuitry is further coupled to a wireless interfacing circuitry 50 and configured to forward the measurements to the wireless interfacing circuitry 50. Wireless interface 50 may support a short range and/or low power wireless communication protocol for efficient transmission of the measurements to a receiving part of the system. Wireless interface 50 may for example operate according to the Bluetooth Low Energy, BLE, protocol as defined by the Bluetooth Special Interest Group or according to a Near Field Communication, NFC, protocol. Operation by such protocols together with forwarding of the raw optical plethysmography signal 101 allows miniaturization of the apparatus 10 such that it fits on a finger or a nostril and allows operation during multiple nights. According to an alternative embodiment, the apparatus 10 obtains one or more of the optical plethysmography signal 101, light intensities 102;103, the oxygen saturation estimate 104, the calibration data 105 from the memory 6. According to a further alternative embodiment, the apparatus 10 obtains one or more of the optical plethysmography signal 101, light intensities 102;103, the oxygen saturation estimate 104, the calibration data 105 from the light sources 2;3 and/or the sensor 4 and/or from the memory 6. The calibration data 105 comprises predetermined calibration coefficients 25 and/or predefined coefficients 26. The apparatus 10 is then further configured to determine the compensation function 14 by deriving the compensation function 14 from the predefined coefficients 26. According to an alternative embodiment, the apparatus 10 is configured to determine the compensation function 14 by fitting the oxygen saturation estimate 104 to the predetermined calibration coefficients 25. Optionally, at least one point in time 12 corresponds to the diastole of a cardiac cycle of the individual and/or at least one point in time 13 corresponds to the systole of a cardiac cycle of the individual. The apparatus 10 is configured to determine an evaluation function 17 which is a function of the light intensities 102;103. The apparatus 10 collects, by optical plethysmography on the sensor 4, propagated light 41 corresponding to light 40 emitted by the first light source 2 or by the second light source 3, wherein the light 40 is transmitted or reflected by the investigated volume 11 when propagating in the distal end of the digit of the individual at the two or more points in time 12;13 along the optical plethysmography signal 101. In other words, the apparatus 10 collects, by optical plethysmography on the sensor 4, a light intensity 102 corresponding to propagated light 41 corresponding to light 40 emitted by the first light source 2 or by the second light source 3, wherein the light 40 is transmitted or reflected by the investigated volume 11 when propagating in the distal end of the digit of the individual 1 and is collected on the sensor 4 at a first point in time 12; and the apparatus 10 collects, by optical plethysmography on the sensor 4, a light intensity 103 corresponding to propagated light 41 corresponding to light 40 emitted by the same light source 2 or 3, wherein the light 40 is transmitted or reflected by the investigated volume 11 when propagating in the distal end of the digit of the individual 1 and is collected on the sensor 4 at a second point in time 13. The apparatus then determines a ratio 15 corresponding to a ratio of the evaluation function 17 and of the compensation function 14. The evaluation function 17 for example corresponds to a logarithm of a function of the light intensities 102;103. The evaluation function 17 for example corresponds to a logarithm of a ratio of the light intensities 102;103. The evaluation function 17 depends on one or more of the following: an optical path length, a function of the oxygen saturation estimate, a change in arterial blood volume in the investigated volume. The apparatus 101 optionally determines the oxygen saturation estimate 104 near the distal end of the digit of the individual 1. Evaluating the compensation function 14 optionally corresponds to determining a regression which maps the oxygen saturation estimate 104 onto the predetermined calibration coefficients 25. The apparatus 10 optionally forces that the regression uses a first-degree rational mapping when evaluating the compensation function 14.

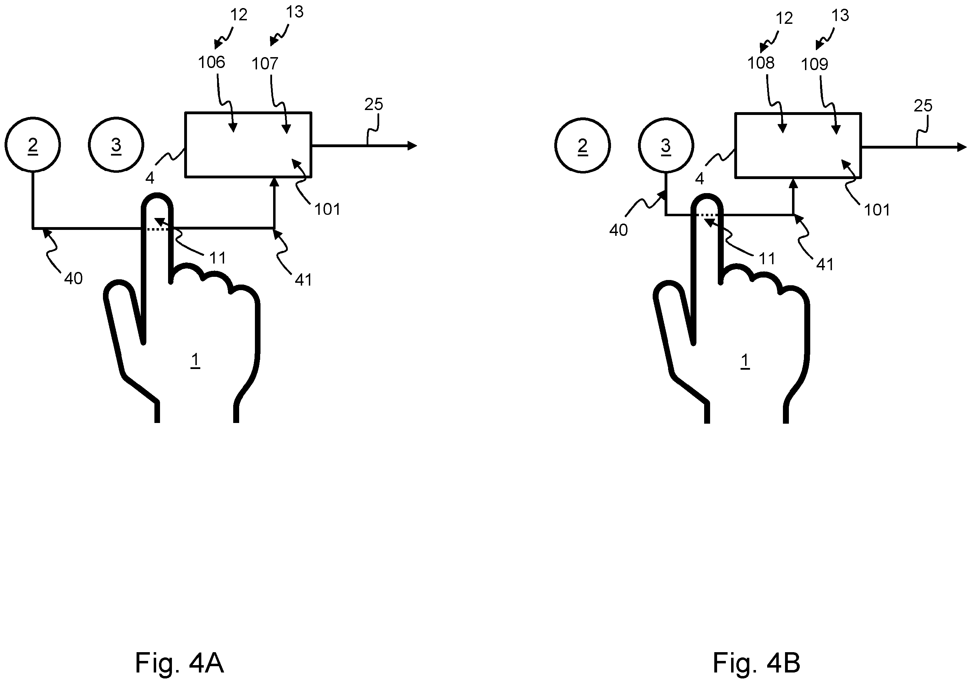

Figs. 4A and 4B illustrate an example embodiment of the calibration of the apparatus according to the present disclosure. Components having identical reference numbers to numbers on Fig. 1 or Fig. 2 or Fig. 3 perform the same function. A first light source 2 is provided and emits light 40 at a first wavelength. A second light source 3 is provided and emits light 40 at a second wavelength. The second wavelength is preferable different from the first wavelength. A sensor 4 is provided. By optical plethysmography, propagated light 41 is collected on the sensor 4 at two points in time 2;3, wherein the propagated light 41 corresponds to the light 40 emitted by the first light source 2 or by the second light source 3 being transmitted or reflected by the investigated volume of an individual when propagating in the investigated volume 11 of the individual 1. As depicted on Fig. 4A, for the first wavelength emitted by the light source 2, light intensities 106;107 corresponding to the propagated light 41 collected on the sensor 4 are measured by the sensor 4 at respectively a first point in time 12 and at a second point in time 13. As depicted on Fig. 4B, for the second wavelength emitted by the second light source 3, light intensities 108;109 corresponding to the propagated light 41 collected on the sensor 4 are measured by the sensor 4 at respectively a first point in time 12 and at a second point in time 13. The sensor 4 or, according to an alternative embodiment the apparatus 10 of Fig. 1 or Fig. 2 or Fig. 3, then determines a first ratio corresponding to a ratio of the first light intensities 106;107 for the first wavelength on Fig. 4A. The sensor 4 or, according to an alternative embodiment the apparatus 10 of Fig. 1 or Fig. 2 or Fig. 3, then determines a second ratio corresponding to a ratio of the second light intensities 108;108 for the second wavelength on Fig. 4B. The sensor 4 or, according to an alternative embodiment the apparatus 10 of Fig. 1 or Fig. 2 or Fig. 3, then determines a calibration ratio corresponding to the ratio of a function of the first ratio and of a function of the second ratio. The sensor 4 or, according to an alternative embodiment the apparatus 10 of Fig. 1 or Fig. 2 or Fig. 3, then determines the predetermined calibration coefficients by fitting the oxygen saturation estimate to the calibration ratio. According to a further alternative embodiment, the determination of the first ratio and/or of the second ratio and/or of the calibration ratio and/or of the predetermined calibration coefficients is performed by any other suitable external device comprising at least one processor and at least one memory including computer program code, the at least one memory and computer program code configured to, with the at least one processor, cause the suitable external device to determine these values.

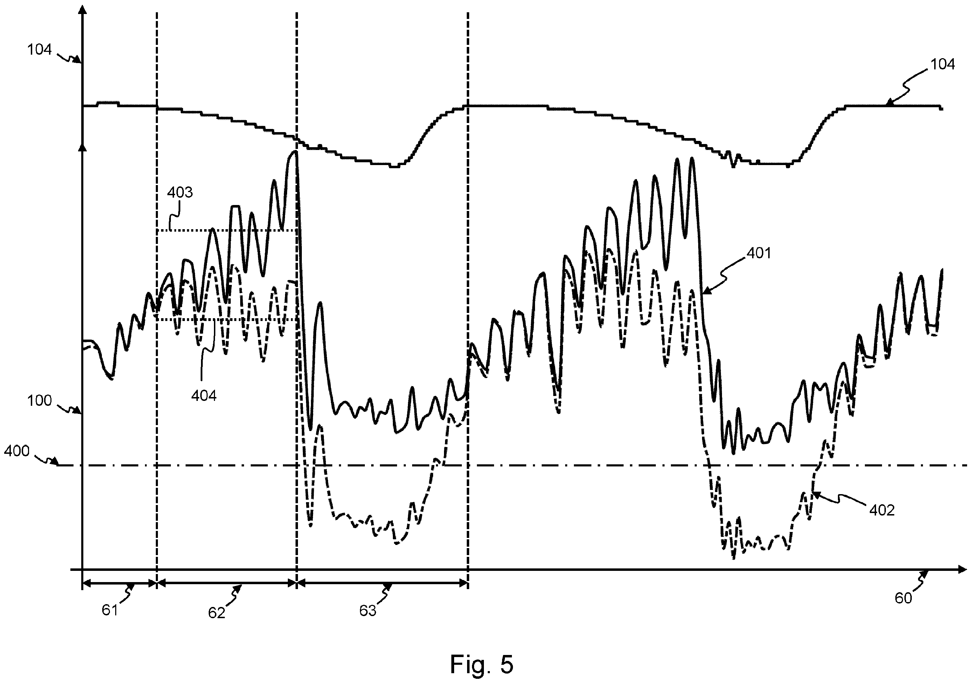

Fig. 5 illustrates an example comparison between uncompensated peripheral arterial tone 401 in function of time 60 of an individual, wherein the uncompensated peripheral arterial tone 401 is determined with optical plethysmography without compensation of the influence caused by changes in the hemoglobin composition of the monitored arterial blood volume on the measurement of peripheral arterial tone, and compensated peripheral arterial tone 402 in function of time 60 of the same individual, wherein the compensated peripheral arterial tone 402 is determined by the computer-implemented method according to the present disclosure, or by the apparatus according to the present disclosure, i.e. wherein the compensated peripheral arterial tone 402 is compensated of the influence caused by changes in the hemoglobin composition of the monitored arterial blood volume on the measurement of peripheral arterial tone. For clarity reasons, the peripheral arterial tone estimates are plotted by calculating $ln\left(\frac{I_h}{I_l}\right)$. According to an alternative embodiment, the peripheral arterial tone estimates are plotted by calculating $ln\left(\frac{I_l}{I_h}\right)$. Fig. 5 also illustrates an example embodiment of the oxygen saturation estimate 104 measured at the same time as the investigated tone 100. As can be seen on Fig. 5, during the time period 61, i.e. during the pre-vasoconstriction-event period 61, the level of oxygen saturation estimate 104 is high and the uncompensated peripheral arterial tone 401 and the compensated peripheral arterial tone 402 evolve in a similar manner and overlap as a function of time 60 at a pre-vasoconstriction-event baseline value. During the time period 62, the value of the oxygen saturation estimate 104 slowly drops, as an indication of an occurrence of an event at the individual under monitoring such as for example a sleep-related event, such as for example sleep apnea. It can be seen on Fig. 5 that the uncompensated peripheral arterial tone 401 and the compensated peripheral arterial tone 402 evolve in a similar manner but do not overlap anymore in the corresponding time period 62. Indeed, the average uncompensated baseline 403 around which the uncompensated peripheral arterial tone 401 evolves during the time period 62 is larger than the average compensated baseline 404 around which the compensated peripheral arterial tone 402 evolves. This clearly shows the effect of the compensation of the influence caused by changes in the hemoglobin composition of the monitored arterial blood volume on the measurement of peripheral arterial tone. During the time period 63, the value of the oxygen saturation estimate 104 reaches its slowest point, corresponding to the occurrence of an event at the individual under monitoring such as for example a sleep-related event, such as for example sleep apnea. It can be seen on Fig. 5 that the uncompensated peripheral arterial tone 401 and the compensated peripheral arterial tone 402 evolve in a similar manner but that compensating for the influence caused by changes in the hemoglobin composition of the monitored arterial blood volume on the measurement of peripheral arterial tone 100 allows to more accurately detect the event occurring. Indeed, a drop in peripheral arterial tone 100 is indicative for a vasoconstriction of the arteries and the arterioles in the investigated volume under monitoring. This vasoconstriction-event can be related to the occurrence of an event at the individual under monitoring such as for example a sleep-related event, such as for example sleep apnea. As it can be seen on Fig. 5, the drop in the uncompensated peripheral arterial tone 402 between the pre-vasoconstriction-event baseline value and the lowest point of the uncompensated peripheral arterial tone 402 is smaller than the drop in the compensated peripheral arterial tone 401 between the pre-vasoconstriction-event baseline value and the lowest point of the compensated peripheral arterial tone 401. For example, a predetermined threshold value 400 for peripheral arterial tone 100 can be used to detect whether an event is occurring at the individual under monitoring such as for example a sleep-related event, such as for example sleep apnea: when the peripheral arterial tone 100 is above this predetermined threshold value 400, no event is detected, but when the peripheral arterial tone 100 is below this predetermined threshold value 400, an event is detected. The drop in the uncompensated peripheral arterial tone 402 between the pre-vasoconstriction-event baseline value and the lowest point of the uncompensated peripheral arterial tone 402 is such that the uncompensated peripheral arterial tone 402 stays above the predetermined threshold value 400, which results in the absence of detection of an event occurring at the individual under monitoring such as for example a sleep-related event, such as for example sleep apnea. On the other hand, the drop in the compensated arterial tone 401 between the pre-vasoconstriction-event baseline value and the lowest point of the compensated peripheral arterial tone 401 is such that the compensated peripheral arterial tone 401 drops below the predetermined threshold value 400, which results in the detection of an event occurring at the individual under monitoring such as for example a sleep-related event, such as for example sleep apnea. It can therefore be seen that compensating the influence caused by changes in the hemoglobin composition of the monitored arterial blood volume on the measurement of peripheral arterial tone 100 allows to more accurately and more robustly detect the occurrence of an event occurring at the individual under monitoring such as for example a sleep-related event, such as for example sleep apnea.

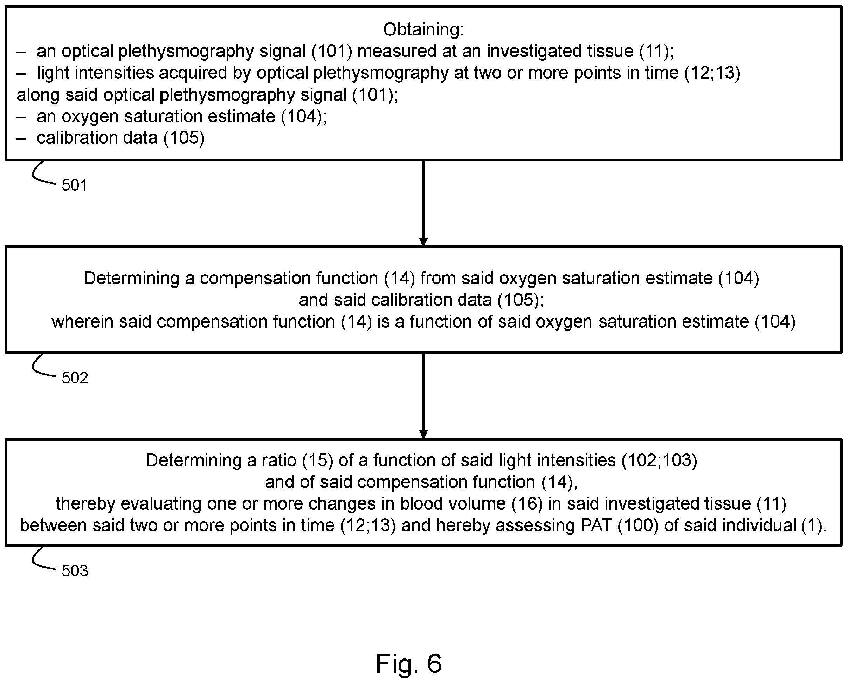

Fig. 6 illustrates an example embodiment of a computer-implemented method for assessing peripheral arterial tone, PAT, of an individual monitored by optical plethysmography, wherein said method comprises the steps of:

- in a first step 501, obtaining:

  ○ an optical plethysmography signal measured at an investigated volume of said individual;
  ○ light intensities acquired by optical plethysmography at two or more points in time along said optical plethysmography signal;
  ○ an oxygen saturation estimate;
  ○ calibration data;

- in a second step 502 consecutive to the first step 501, determining a compensation function from said oxygen saturation estimate and said calibration data; wherein said compensation function is a function of said oxygen saturation estimate; and determining a first function of said light intensities; and
- in a third step 503 consecutive to the second step 502, determining a ratio of said function and said compensation function, thereby evaluating one or more changes in arterial blood volume in said investigated volume between said two or more points in time and hereby assessing PAT of said individual.

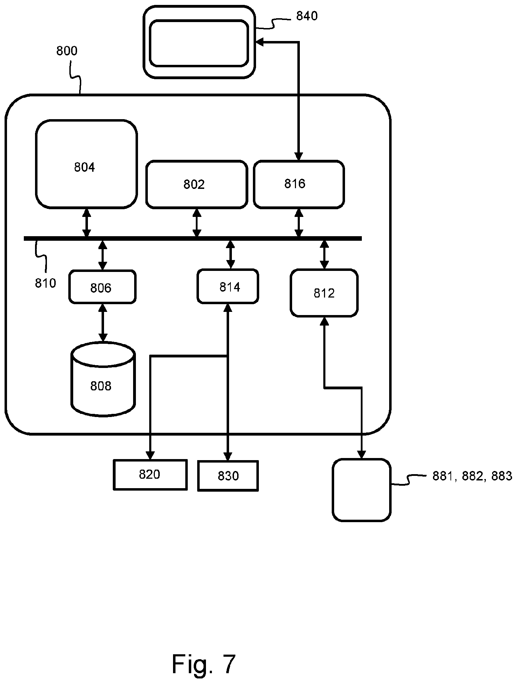

Fig. 7 shows a suitable computing system 800 enabling to implement embodiments of the system. Computing system 800 may in general be formed as a suitable general-purpose computer and comprise a bus 810, a processor 802, a local memory 804, one or more optional input interfaces 814, one or more optional output interfaces 816, a communication interface 812, a storage element interface 806, and one or more storage elements 808. Bus 810 may comprise one or more conductors that permit communication among the components of the computing system 800. Processor 802 may include any type of conventional processor or microprocessor that interprets and executes programming instructions. Local memory 804 may include a random-access memory (RAM) or another type of dynamic storage device that stores information and instructions for execution by processor 802 and/or a read only memory (ROM) or another type of static storage device that stores static information and instructions for use by processor 802. Input interface 814 may comprise one or more conventional mechanisms that permit an operator or user to input information to the computing device 800, such as a keyboard 820, a mouse 830, a pen, voice recognition and/or biometric mechanisms, a camera, etc. Output interface 816 may comprise one or more conventional mechanisms that output information to the operator or user, such as a display 840, etc. Communication interface 812 may comprise any transceiver-like mechanism such as for example one or more Ethernet interfaces that enables computing system 800 to communicate with other devices and/or systems, for example with other computing devices 881, 882, 883. The communication interface 812 of computing system 800 may be connected to such another computing system by means of a local area network (LAN) or a wide area network (WAN) such as for example the internet. Storage element interface 806 may comprise a storage interface such as for example a Serial Advanced Technology Attachment (SATA) interface or a Small Computer System Interface (SCSI) for connecting bus 810 to one or more storage elements 808, such as one or more local disks, for example SATA disk drives, and control the reading and writing of data to and/or from these storage elements 808. Although the storage element(s) 808 above is/are described as a local disk, in general any other suitable computer-readable media such as a removable magnetic disk, optical storage media such as a CD or DVD, -ROM disk, solid state drives, flash memory cards, ... could be used. Computing system 800 could thus correspond to the apparatus 10 in the embodiment illustrated by Figs. 1 or 2 or 3.

As used in this application, the term "circuitry" may refer to one or more or all of the following:

(a) hardware-only circuit implementations such as implementations in only analog and/or digital circuitry and
(b) combinations of hardware circuits and software, such as (as applicable):

  (i) a combination of analog and/or digital hardware circuit(s) with software/firmware and

(ii) any portions of hardware processor(s) with software (including digital signal processor(s)), software, and memory(ies) that work together to cause an apparatus, such as a mobile phone or server, to perform various functions) and

(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

The invention is defined by the appended claims.

## Claims

1. A computer-implemented method for assessing peripheral arterial tone (100), PAT, of an individual (1) monitored by optical plethysmography, wherein said method comprises the steps of:

   - obtaining:

     ∘ an optical plethysmography signal (101) measured at an investigated volume (11) of said individual (1);
     ∘ light intensities (102;103) acquired by optical plethysmography at two or more points in time (12;13) along said optical plethysmography signal (101);
     ∘ an oxygen saturation estimate (104);
     ∘ calibration data (105);

   - determining a compensation function (14) from said oxygen saturation estimate (104) and said calibration data (105); wherein said compensation function (14) is a function of said oxygen saturation estimate (104);
   - determining a first function of said light intensities (102;103); and
   - determining a ratio (15) of the first function and said compensation function (14), thereby evaluating changes in arterial blood volume (16) in said investigated volume (11) between said two or more points in time (12;13) and hereby assessing PAT (100) of said individual (1).

2. The method according to claim 1, wherein said calibration data (105) comprises predetermined calibration coefficients (25) and/or predefined coefficients (26); and wherein:

   - said determining said compensation function (14) corresponds to deriving said compensation function (14) from said predefined coefficients (26); or
   - said determining said compensation function (14) corresponds to determining said predetermined calibration coefficients (25) by fitting said oxygen saturation estimate (104) to a calibration ratio.

3. The method according to claim 1 or 2, wherein at least one (12) of said points in time (12;13) corresponds to the diastole in a cardiac cycle of said individual (1) and/or wherein at least one (13) of said points in time (12;13) corresponds to the systole in a cardiac cycle of said individual (1).

4. The method according to any of the preceding claims, wherein said method further comprises the steps of determining an evaluation function (17) which is a function of said light intensities (102;103); and wherein said determining a ratio (15) corresponds to determining a ratio of said evaluation function (17) and said compensation function (14).

5. The method according to claim 4, wherein said evaluation function (17) corresponds to a logarithm of a function of said light intensities (102;103).

6. The method according to claim 4 or 5, wherein said evaluation function (17) corresponds to a logarithm of a ratio of said light intensities (102;103); and wherein said evaluation function (17) depends on one or more of the following:

- a function of said oxygen saturation estimate (104);
- said changes in arterial blood volume (16) in said investigated volume (11).

7. The method according to any of the preceding claims, wherein said method further comprises the steps of:

- providing a first light source (2) configured to emit light at a first wavelength;
- providing a second light source (3) configured to emit light at a second wavelength;
- providing a sensor (4);
- collecting, by optical plethysmography and on said sensor (4), propagated light corresponding to said light being transmitted or reflected when propagating in said investigated volume (11) of said individual (1) at said two or more points (12;13) in time;
- for said first wavelength, determining first light intensities (106;107) of said propagated light on said sensor (4) at said two or more points in time (12;13);
- for said second wavelength, determining second light intensities (108;109) of said propagated light on said sensor (4) at said two or more points in time (12;13);
- determining a first ratio corresponding to a ratio of said first light intensities (106;107) at said first wavelength;
- determining a second ratio corresponding to a ratio of said second light intensities (108;109) at said second wavelength;
- determining said calibration ratio of a function of said first ratio and of a function of said second ratio; and
- fitting said oxygen saturation estimate (104) to said calibration ratio thereby determining said predetermined calibration coefficients (25).

8. The method according to claim 7, wherein said method further comprises the steps of:

- collecting, by optical plethysmography and on said sensor (4), propagated light (41) corresponding to light (40) at said first wavelength or at said second wavelength being transmitted or reflected when propagating in said investigated volume (11) of said individual (1) at said two or more points in time (12;13); and
- determining said light intensities (102;103) of said propagated light on said sensor (4) at said two or more points in time (12;13).

9. The method according to claim 7 or 8, wherein said method further comprises the step of determining said oxygen saturation estimate (104) in the vicinity of said investigated volume (11) of said individual (1).

10. The method according to any of the preceding claims, wherein said compensation function (14) is derived from a regression which maps said oxygen saturation estimate (104) onto said predetermined calibration coefficients (25).

11. The method according to claim 10, wherein said method further comprises the step of forcing that said regression uses a first-degree rational mapping when evaluating said compensation function (14).

12. An apparatus (10) comprising at least one processor and at least one memory (6) including computer program code, the at least one memory (6) and computer program code configured to, with the at least one processor, cause the apparatus (10) to perform:

- obtaining:

  ◦ an optical plethysmography signal (101) measured at an investigated volume (11) of an individual (1);
  ◦ light intensities (102;103) acquired by optical plethysmography at two or more points in time (12;13) along said optical plethysmography signal (101);
  ◦ an oxygen saturation estimate (104);
  ◦ calibration data (105);

- determining a compensation function (14) from said oxygen saturation estimate (104) and said calibration data (105); wherein said compensation function (14) is a function of said oxygen saturation estimate (104);
- determining a first function of said light intensities (102;103); and
- determining a ratio (15) of the first function and said compensation function (14), thereby evaluating changes in arterial blood volume (16) in said investigated volume (11) between said two or more points in time (12;13) and hereby assessing PAT (100) of said individual (1).

13. A system (20) comprising an apparatus according to claim 12, and further comprising:

- a light source (2;3) configured to emit light; and
- a sensor (4) configured to collect by optical plethysmography propagated light corresponding to said light being transmitted or reflected when propagating in a distal end of a digit of said individual (1) at said two or more points in time (12;13); and further configured to determine said light intensities (102;103) of said propagated light at said two or more points in time (12;13).

14. A computer readable storage medium comprising computer-executable instructions for performing the following steps when the program is run on a computer:

- obtaining:

  ∘ an optical plethysmography signal (101) measured at an investigated volume (11) of an individual (1);
  ∘ light intensities (102;103) acquired by a sensor (4) of optical plethysmography at two or more points in time (12;13) along said optical plethysmography signal (101);
  ∘ an oxygen saturation estimate (104);
  ∘ calibration data (105);

- determining a compensation function (14) from said oxygen saturation estimate (104) and said calibration data (105); wherein said compensation function (14) is a function of said oxygen saturation estimate (104);
- determining a first function of said light intensities (102;103); and
- determining a ratio (15) of the first function and said compensation function (14), thereby evaluating changes in arterial blood volume (16) in said investigated volume (11) between said two or more points in time (12;13) and hereby assessing PAT (100) of said individual (1).

**Patentansprüche**

1. Computerimplementiertes Verfahren zur Beurteilung des peripheren arteriellen Tonus, PAT, (100) eines Individuums (1), der durch optische Plethysmographie überwacht wird, wobei das Verfahren umfasst:

- Erhalten:

  - eines optischen Plethysmographiesignals (101), das an einem untersuchten Volumen (11) des Individuums (1) durch einen Sensor einer Anordnung für optische Plethysmographie gemessen wird; und
  - von Lichtintensitäten (102; 103), die durch optische Plethysmographie zu zwei oder mehr Zeitpunkten (12; 13) entlang des optischen Plethysmographiesignals (101) erfasst werden; und
  - eine Schätzung der Sauerstoffsättigung (104);
  - von Kalibrierungsdaten (105);

- Bestimmen einer Kompensationsfunktion (14) aus der Schätzung der Sauerstoffsättigung (104) und den Kalibrierungsdaten (105); wobei die Kompensationsfunktion (14) eine Funktion der Schätzung der Sauerstoffsättigung (104) ist;
- Bestimmen einer ersten Funktion der Lichtintensitäten (102; 103); und
- Bestimmen eines Verhältnisses (15) der ersten Funktion und der Kompensationsfunktion (14), wodurch Veränderungen im arteriellen Blutvolumen (16) in dem untersuchten Volumen (11) zwischen den zwei oder mehr Zeitpunkten (12; 13) evaluiert und dadurch der PAT (100) des Individuums (1) beurteilt werden.

2. Verfahren nach Anspruch 1, wobei die Kalibrierungsdaten (105) vorbestimmte Kalibrierungskoeffizienten (25) und/oder vorbestimmte Koeffizienten (26) umfassen; und wobei:

- das Bestimmen der Kompensationsfunktion (14) dem Ableiten der Kompensationsfunktion (14) von den vorbestimmten Koeffizienten (26) entspricht; oder
- das Bestimmen der Kompensationsfunktion (14) dem Bestimmen der vorbestimmten Kalibrierungskoeffizienten (25) durch Anpassen der Schätzung der Sauerstoffsättigung (104) an ein Kalibrierungsverhältnis entspricht.

3. Verfahren nach Anspruch 1 oder 2, wobei mindestens einer (12) der Zeitpunkte (12; 13) der Diastole in einem Herzzyklus des Individuums (1) entspricht und/oder wobei mindestens einer (13) der Zeitpunkte (12; 13) der Systole in einem Herzzyklus des Individuums (1) entspricht.

4. Verfahren nach einem der vorhergehenden Ansprüche, wobei das Verfahren ferner die Schritte des Bestimmens einer Evaluierungsfunktion (17) umfasst, die eine Funktion der Lichtintensitäten (102; 103) ist; und wobei das Bestimmen eines Verhältnisses (15) dem Bestimmen eines Verhältnisses der Evaluierungsfunktion (17) und der Kompensationsfunktion (14) entspricht.

5. Verfahren nach Anspruch 4, wobei die Evaluierungsfunktion (17) einem Logarithmus einer Funktion der Lichtintensitäten (102; 103) entspricht.

6. Verfahren nach Anspruch 4 oder 5, wobei die Evaluierungsfunktion (17) einem Logarithmus eines Verhältnisses der Lichtintensitäten (102, 103) entspricht; und wobei die Evaluierungsfunktion (17) von einem oder mehreren aus Folgendem abhängig ist:

   - einer Funktion der Schätzung der Sauerstoffsättigung (104);
   - den Veränderungen im arteriellen Blutvolumen (16) in dem untersuchten Volumen (11).

7. Verfahren nach einem der vorhergehenden Ansprüche, wobei das Verfahren ferner die folgenden Schritte umfasst:

   - Bereitstellen einer ersten Lichtquelle (2), die dazu ausgelegt ist, Licht mit einer ersten Wellenlänge zu emittieren;
   - Bereitstellen einer zweiten Lichtquelle (3), die dazu ausgelegt ist, Licht mit einer zweiten Wellenlänge zu emittieren;
   - Bereitstellen eines Sensors (4);
   - Erfassen, durch optische Plethysmographie und an dem Sensor (4), von sich ausbreitendem Licht, das dem Licht entspricht, das bei der Ausbreitung in dem untersuchten Volumen (11) des Individuums (1) zu den zwei oder mehr Zeitpunkten (12; 13) durchgelassen oder reflektiert wird; und
   - Bestimmen der Lichtintensitäten (106; 107) des sich ausbreitenden Lichts an dem Sensor (4) zu den zwei oder mehr Zeitpunkten (12; 13) für die erste Wellenlänge;
   - Bestimmen der Lichtintensitäten (108; 109) des sich ausbreitenden Lichts an dem Sensor (4) zu den zwei oder mehr Zeitpunkten (12; 13) für die zweite Wellenlänge;
   - Bestimmen eines ersten Verhältnisses entsprechend einem Verhältnis der ersten Lichtintensitäten (106; 107) mit der ersten Wellenlänge;
   - Bestimmen eines zweiten Verhältnisses entsprechend einem Verhältnis der zweiten Lichtintensitäten (108; 109) mit der zweiten Wellenlänge;
   - Bestimmen des Kalibrierungsverhältnisses einer Funktion des ersten Verhältnisses und einer Funktion des zweiten Verhältnisses; und
   - Anpassen der Schätzung der Sauerstoffsättigung (104) an das Kalibrierungsverhältnis und dadurch Bestimmen der vorbestimmten Kalibrierungskoeffizienten (25).

8. Verfahren nach Anspruch 7, wobei das Verfahren ferner die folgenden Schritte umfasst:

   - Erfassen, durch optische Plethysmographie und an dem Sensor (4), von sich ausbreitendem Licht (41), das dem Licht (40) mit der ersten Wellenlänge oder mit der zweiten Wellenlänge entspricht, das bei der Ausbreitung im untersuchten Volumen (11) des Individuums (1) zu den zwei oder mehr Zeitpunkten (12; 13) durchgelassen oder reflektiert wird; und
   - Bestimmen der Lichtintensitäten (102; 103) des sich ausbreitenden Lichts am Sensor (4) zu den zwei oder mehr Zeitpunkten (12; 13).

9. Verfahren nach Anspruch 7 oder 8, wobei das Verfahren ferner den Schritt des Bestimmens der Schätzung der Sauerstoffsättigung (104) in der Umgebung des untersuchten Volumens (11) des Individuums (1) umfasst.

10. Verfahren nach einem der vorhergehenden Ansprüche, wobei die Kompensationsfunktion (14) von einer Regression abgeleitet ist, welche die Schätzung der Sauerstoffsättigung (104) auf die vorbestimmten Kalibrierungskoeffizienten (25) abbildet.

11. Verfahren nach Anspruch 10, wobei das Verfahren ferner den Schritt des Erzwingens umfasst, dass die Regression eine rationale Abbildung ersten Grades verwendet, wenn die Kompensationsfunktion (14) evaluiert wird.

12. Vorrichtung (10), umfassend mindestens einen Prozessor und mindestens einen Speicher (6), der Computerprogrammcode beinhaltet, wobei der mindestens eine Speicher (6) und der Computerprogrammcode dazu ausgelegt sind, mit dem mindestens einen Prozessor die Vorrichtung (10) zu veranlassen,

- zu erhalten:

- ein optisches Plethysmographiesignal (101), das an einem untersuchten Volumen (11) des Individuums (1) durch einen Sensor einer Anordnung für optische Plethysmographie gemessen wird; und
- Lichtintensitäten (102; 103), die durch optische Plethysmographie zu zwei oder mehr Zeitpunkten (12; 13) entlang des optischen Plethysmographiesignals (101) erfasst werden; und
- eine Schätzung der Sauerstoffsättigung (104);
- Kalibrierungsdaten (105);

- eine Kompensationsfunktion (14) aus der Schätzung der Sauerstoffsättigung (104) und den Kalibrierungsdaten (105) zu bestimmen; wobei die Kompensationsfunktion (14) eine Funktion der Schätzung der Sauerstoffsättigung (104) ist;
- eine erste Funktion der Lichtintensitäten (102; 103) zu bestimmen; und
- ein Verhältnis (15) der ersten Funktion und der Kompensationsfunktion (14) zu bestimmen, wodurch Veränderungen im arteriellen Blutvolumen (16) in dem untersuchten Volumen (11) zwischen den zwei oder mehr Zeitpunkten (12; 13) evaluiert und dadurch der PAT (100) des Individuums (1) beurteilt werden.

13. System (20), umfassend eine Vorrichtung nach Anspruch 12, und ferner umfassend:

- eine Lichtquelle (2; 3), die dazu ausgelegt ist, Licht zu emittieren, und
- einen Sensor (4), der so ausgelegt ist, dass er sich ausbreitendes Licht, das dem Licht entspricht, das bei Ausbreitung in einem distalen Ende eines Fingers des Individuums (1) zu den zwei oder mehr Zeitpunkten (12; 13) durchgelassen oder reflektiert wird, durch optische Plethysmographie erfasst; und ferner so ausgelegt ist, dass er die Lichtintensitäten (102; 103) des sich ausbreitenden Lichts zu den zwei oder mehr Zeitpunkten (12; 13) bestimmt.

14. Computerlesbares Speichermedium, umfassend computerausführbare Anweisungen zum Durchführen des Verfahrens der folgenden Schritte, wenn das Programm auf einem Computer ausgeführt wird:

- Erhalten:

- eines optischen Plethysmographiesignals (101), das an einem untersuchten Volumen (11) eines Individuums (1) gemessen wird; und
- von Lichtintensitäten (102; 103), die durch einen Sensor (4) für die optische Plethysmographie zu zwei oder mehr Zeitpunkten (12; 13) entlang des optischen Plethysmographiesignals (101) erfasst werden; und
- einer Schätzung der Sauerstoffsättigung (104);
- von Kalibrierungsdaten (105);

- Bestimmen einer Kompensationsfunktion (14) aus der Schätzung der Sauerstoffsättigung (104) und den Kalibrierungsdaten (105); wobei die Kompensationsfunktion (14) eine Funktion der Schätzung der Sauerstoffsättigung (104) ist;
- Bestimmen einer ersten Funktion der Lichtintensitäten (102; 103); und
- Bestimmen eines Verhältnisses (15) der ersten Funktion und der Kompensationsfunktion (14), wodurch Veränderungen im arteriellen Blutvolumen (16) in dem untersuchten Volumen (11) zwischen den zwei oder mehr Zeitpunkten (12; 13) evaluiert und dadurch der PAT (100) des Individuums (1) beurteilt werden.

**Revendications**

1. Procédé mis en oeuvre par ordinateur pour évaluer le tonus artériel périphérique (100), PAT, d'un individu (1) surveillé par pléthysmographie optique, dans lequel ledit procédé comprend les étapes :

- d'obtention :

- d'un signal de pléthysmographie optique (101) mesuré à un volume étudié (11) dudit individu (1) ;
- d'intensités lumineuses (102 ; 103) acquises par pléthysmographie optique à deux instants ou plus (12 ; 13) le long dudit signal de pléthysmographie optique (101) ;
- d'une estimation de saturation en oxygène (104) ;
- de données d'étalonnage (105) ;

- de détermination d'une fonction de compensation (14) à partir de ladite estimation de saturation en oxygène (104) et desdites données d'étalonnage (105) ; dans lequel ladite fonction de compensation (14) est fonction de ladite estimation de saturation en oxygène (104) ;
- de détermination d'une première fonction desdites intensités lumineuses (102 ; 103) ; et
- de détermination d'un rapport (15) de la première fonction et de ladite fonction de compensation (14), évaluant ainsi les changements du volume sanguin artériel (16) dans ledit volume étudié (11) entre lesdits deux instants ou plus (12 ; 13) et évaluant ainsi le PAT (100) dudit individu (1).

2. Procédé selon la revendication 1, dans lequel lesdites données d'étalonnage (105) comprennent des coefficients d'étalonnage prédéterminés (25) et/ou des coefficients prédéfinis (26) ; et dans lequel :

- ladite détermination de ladite fonction de compensation (14) correspond à la dérivation de ladite fonction de compensation (14) desdits coefficients prédéfinis (26) ; ou
- ladite détermination de ladite fonction de compensation (14) correspond à la détermination desdits coefficients d'étalonnage prédéterminés (25) en ajustant ladite estimation de saturation en oxygène (104) à un rapport d'étalonnage.

3. Procédé selon la revendication 1 ou 2, dans lequel au moins un (12) desdits instants (12 ; 13) correspond à la diastole dans un cycle cardiaque dudit individu (1) et/ou dans lequel au moins un (13) desdits instants (12 ; 13) correspond à la systole dans un cycle cardiaque dudit individu (1).

4. Procédé selon l'une des revendications précédentes, dans lequel ledit procédé comprend en outre les étapes de détermination d'une fonction d'évaluation (17) qui est fonction desdites intensités lumineuses (102 ; 103) ; et dans lequel ladite détermination d'un rapport (15) correspond à la détermination d'un rapport de ladite fonction d'évaluation (17) et de ladite fonction de compensation (14).

5. Procédé selon la revendication 4, dans lequel ladite fonction d'évaluation (17) correspond à un logarithme d'une fonction desdites intensités lumineuses (102 ; 103).

6. Procédé selon la revendication 4 ou 5, dans lequel ladite fonction d'évaluation (17) correspond à un logarithme d'un rapport desdites intensités lumineuses (102 ; 103) ; et dans lequel ladite fonction d'évaluation (17) dépend d'un ou de plusieurs de ce qui suit :

- une fonction de ladite estimation de saturation en oxygène (104) ;
- lesdits changements du volume sanguin artériel (16) dans ledit volume étudié (11).

7. Procédé selon l'une des revendications précédentes, dans lequel ledit procédé comprend en outre les étapes :

- de fourniture d'une première source de lumière (2) configurée pour émettre de la lumière à une première longueur d'onde ;
- de fourniture d'une deuxième source de lumière (3) configurée pour émettre de la lumière à une seconde longueur d'onde ;
- de fourniture d'un capteur (4) ;
- de collecte, par pléthysmographie optique et sur ledit capteur (4), de la lumière propagée correspondant à ladite lumière qui est transmise ou réfléchie lors de sa propagation dans ledit volume étudié (11) dudit individu (1) auxdits deux instants ou plus (12 ; 13) ;
- pour ladite première longueur d'onde, de détermination de premières intensités lumineuses (106 ; 107) de ladite lumière propagée sur ledit capteur (4) auxdits deux instants ou plus (12 ; 13) ;
- pour ladite seconde longueur d'onde, de détermination de secondes intensités lumineuses (108 ; 109) de ladite lumière propagée sur ledit capteur (4) auxdits deux instants ou plus (12 ; 13) ;
- de détermination d'un premier rapport correspondant à un rapport desdites premières intensités lumineuses (106 ; 107) à ladite première longueur d'onde ;

- de détermination d'un second rapport correspondant à un rapport desdites secondes intensités lumineuses (108 ; 109) à ladite seconde longueur d'onde ;
- de détermination dudit rapport d'étalonnage d'une fonction dudit premier rapport et d'une fonction dudit second rapport ; et
- d'ajustement de ladite estimation de saturation en oxygène (104) audit rapport d'étalonnage, déterminant ainsi lesdits coefficients d'étalonnage prédéterminés (25).

8. Procédé selon la revendication 7, dans lequel ledit procédé comprend en outre les étapes :

- de collecte, par pléthysmographie optique et sur ledit capteur (4), de la lumière propagée (41) correspondant à la lumière (40) à ladite première longueur d'onde ou à ladite seconde longueur d'onde qui est transmise ou réfléchie lors de sa propagation dans ledit volume étudié (11) dudit individu (1) auxdits deux instants ou plus (12 ; 13) ; et
- de détermination desdites intensités lumineuses (102 ; 103) de ladite lumière propagée sur ledit capteur (4) auxdits deux instants ou plus (12 ; 13).

9. Procédé selon la revendication 7 ou 8, dans lequel ledit procédé comprend en outre l'étape de détermination de ladite estimation de saturation en oxygène (104) à proximité dudit volume étudié (11) dudit individu (1).

10. Procédé selon l'une des revendications précédentes, dans lequel ladite fonction de compensation (14) est dérivée d'une régression qui mappe ladite estimation de saturation en oxygène (104) sur lesdits coefficients d'étalonnage prédéterminés (25).

11. Procédé selon la revendication 10, dans lequel ledit procédé comprend en outre l'étape consistant à forcer ladite régression à utiliser un mappage rationnel du premier degré lors de l'évaluation de ladite fonction de compensation (14).

12. Appareil (10) comprenant au moins un processeur et au moins une mémoire (6) incluant un code de programme informatique, l'au moins une mémoire (6) et le code de programme informatique étant configurés, avec l'au moins un processeur, pour amener l'appareil (10) à réaliser :

- l'obtention :

- d'un signal de pléthysmographie optique (101) mesuré à un volume étudié (11) d'un individu (1) ;
- d'intensités lumineuses (102 ; 103) acquises par pléthysmographie optique à deux instants ou plus (12 ; 13) le long dudit signal de pléthysmographie optique (101) ;
- d'une estimation de saturation en oxygène (104) ;
- de données d'étalonnage (105) ;

- la détermination d'une fonction de compensation (14) à partir de ladite estimation de saturation en oxygène (104) et desdites données d'étalonnage (105) ; dans lequel ladite fonction de compensation (14) est fonction de ladite estimation de saturation en oxygène (104) ;
- la détermination d'une première fonction desdites intensités lumineuses (102 ; 103) ; et
- la détermination d'un rapport (15) de la première fonction et de ladite fonction de compensation (14), évaluant ainsi les changements du volume sanguin artériel (16) dans ledit volume étudié (11) entre lesdits deux instants ou plus (12 ; 13) et évaluant ainsi le PAT (100) dudit individu (1).

13. Système (20) comprenant un appareil selon la revendication 12, et comprenant en outre :

- une source de lumière (2 ; 3) configurée pour émettre de la lumière ; et
- un capteur (4) configuré pour collecter par pléthysmographie optique la lumière propagée correspondant à ladite lumière qui est transmise ou réfléchie lors de sa propagation dans une extrémité distale d'un doigt dudit individu (1) auxdits deux instants ou plus (12 ; 13) ; et configuré en outre pour déterminer lesdites intensités lumineuses (102 ; 103) de ladite lumière propagée auxdits deux instants ou plus (12 ; 13).

14. Support de stockage lisible par ordinateur comprenant des instructions exécutables par ordinateur pour réaliser les étapes suivantes lorsque le programme est exécuté sur un ordinateur :

- d'obtention :

- d'un signal de pléthysmographie optique (101) mesuré à un volume étudié (11) d'un individu (1) ;
- d'intensités lumineuses (102 ; 103) acquises par un capteur (4) de pléthysmographie optique à deux instants ou plus (12 ; 13) le long dudit signal de pléthysmographie optique (101) ;
- d'une estimation de saturation en oxygène (104) ;
- de données d'étalonnage (105) ;

- de détermination d'une fonction de compensation (14) à partir de ladite estimation de saturation en oxygène (104) et desdites données d'étalonnage (105) ; dans lequel ladite fonction de compensation (14) est fonction de ladite estimation de saturation en oxygène (104) ;
- de détermination d'une première fonction desdites intensités lumineuses (102 ; 103) ; et
- de détermination d'un rapport (15) de la première fonction et de ladite fonction de compensation (14), évaluant ainsi les changements du volume sanguin artériel (16) dans ledit volume étudié (11) entre lesdits deux instants ou plus (12 ; 13) et évaluant ainsi le PAT (100) dudit individu (1).

Fig. 1

EP 3 928 689 B1

Fig. 2

EP 3 928 689 B1

Fig. 3

EP 3 928 689 B1

Fig. 4A

Fig. 4B

Fig. 5

Obtaining:
– an optical plethysmography signal (101) measured at an investigated tissue (11);
– light intensities acquired by optical plethysmography at two or more points in time (12;13)
along said optical plethysmography signal (101);
– an oxygen saturation estimate (104);
– calibration data (105)

501

Determining a compensation function (14) from said oxygen saturation estimate (104)
and said calibration data (105);
wherein said compensation function (14) is a function of said oxygen saturation estimate (104)

502

Determining a ratio (15) of a function of said light intensities (102;103)
and of said compensation function (14),
thereby evaluating one or more changes in blood volume (16) in said investigated tissue (11)
between said two or more points in time (12;13) and hereby assessing PAT (100) of said individual (1).

503

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060009700 A1
- US 2019343406 A1
- WO 9804182 A
- EP 1534115 A2

**Non-patent literature cited in the description**

- **CHATTERJEE et al.** Monte Carlo Analysis of Optical Interactions in Reflectance and Transmittance Finger Photoplethysmography. *Sensors (Basel)*, 15 February 2019, vol. 19 (4), 789